# EUROPEAN PATENT SPECIFICATION

(11) **EP 2 210 942 B1**
(45) Date of publication and mention of the grant of the patent: **27.08.2014**
(21) Application number: 08845421.0
(22) Date of filing: 29.10.2008
(51) Int. Cl.: C12N 15/09, C12Q 1/02, C12Q 1/68, G01N 33/15, G01N 33/50

(54) **GENE ASSOCIATED WITH LIVER CANCER, AND METHOD FOR DETERMINATION OF THE RISK OF ACQUIRING LIVER CANCER**
MIT LEBERKREBS ASSOZIIERTES GEN UND VERFAHREN ZUR BESTIMMMUNG DES RISIKOS DER ERKRANKUNG AN LEBERKREBS
GÈNE ASSOCIÉ AU CANCER DU FOIE, ET PROCÉDÉ DE DÉTERMINATION DU RISQUE D'ÊTRE ATTEINT DE CANCER DU FOIE

(30) Priority: 30.10.2007 JP 2007281552
(43) Date of publication of application: 28.07.2010
(73) Proprietor: The University of Tokyo, Bunkyo-ku, Tokyo 113-8654 (JP)
(72) Inventor: ISHIZAWA, Takeaki, Tokyo 113-8654 (JP); KOKUDO, Norihiro, Tokyo 113-8645 (JP); YOSHIKAWA, Hirohide, Tokyo 135-8550 (JP); SHIKAUCHI, Yuko, Tokyo 135-8550 (JP); MURASE, Yacko, Tachikawa-shi, Tokyo 190-0011 (JP)
(74) Representative: Rudolph, Ulrike
(86) International application number: PCT/JP2008/003082
(87) International publication number: WO 2009/057294

(56) References cited:
- KATOH YURIKO ET AL: "Canonical WNT signaling pathway and human AREG" INTERNATIONAL JOURNAL OF MOLECULAR MEDICINE, vol. 17, no. 6, June 2006 (2006-06), pages 1163-1166, XP002603412 ISSN: 1107-3756
- SHIKAUCHI YUKO ET AL: "SALL3 Interacts with DNMT3A and Shows the Ability To Inhibit CpG Island Methylation in Hepatocellular Carcinoma" MOLECULAR AND CELLULAR BIOLOGY, vol. 29, no. 7, April 2009 (2009-04), pages 1944-1958, XP002603413 ISSN: 0270-7306
- DATABASE NUCLEOTIDE [Online] NCBI 28 July 2007 'Homo sapiens sal-like 3 (Drosophila) (SALL3), mRNA.', XP008138294 Database accession no. NM_171999
- DATABASE NUCLEOTIDE NCBI 22 September 2007 'Homo sapiens endothelin converting enzyme-like 1 (ECEL1), mRNA.', XP008138296 Database accession no. NM 004826
- DATABASE NUCLEOTIDE NCBI 25 September 2007 'Homo sapiens forkhead box C1 (FOXC1), mRNA.', XP008138299 Database accession no. NM_001453
- DATABASE NUCLEOTIDE NCBI 26 September 2007 'Homo sapiens neuregulin 3 (NRG3), mRNA.', XP008138595 Database accession no. NM_001010848 & XM_166086
- DATABASE NUCLEOTIDE NCBI 03 September 2007 'Homo sapiens Kv channel interacting protein 2 (KCNIP2), transcript variant 1, mRNA.', XP008138298 Database accession no. NM_014591
- DATABASE NUCLEOTIDE NCBI 29 August 2006 'Homo sapiens chromosome 4 genomic contig, reference assembly.', XP008138596 Database accession no. NT_037622 & NT_006111
- DATABASE NCBI 29 August 2006 'Homo sapiens chromosome 2 genomic contig, reference assembly.', XP008138591 Database accession no. NT_022135 & NT_005011 NT_005058 NT_005060 NT_005079 NT_005112 NT_005138 NT_005445 NT_019306 NT_022176 NT_022199 NT_022222 NT_026982 NT_028085 NT_029250 NT_029923 NT_030592 NT_030595 NT_031759 NT_031762 NT_032990 NT_037534 NT_077999
- ZHAO C. ET AL.: 'Detection of aberrantly methylated genes in hepatocellular carcinoma by BAC-array based MCA(BAMCA) strategy.' THE 66TH JAPANESE CANCER ASSOCIATION GAKUJUTSU SOKAI KIJI August 2007, pages 478 - 1025, XP008142228
- NAGAE G. ET AL.: 'Comprehensive DNA methylation analysis in liver cancer using high-resolution tiling array.' THE 66TH JAPANESE CANCER ASSOCIATION GAKUJUTSU SOKAI KIJI August 2007, pages 312 - 623, XP008142230
- AN BYONGGU ET AL.: 'Genomewide DNA methylation analyses in cancerous and adjacent tissues from hepatocellular carcinoma patients.' THE 66TH JAPANESE CANCER ASSOCIATION GAKUJUTSU SOKAI KIJI August 2007, pages 267 - O.271, XP008142231
- HIROHASHI S.: 'Hito Tadankai Hatsugan Katei ni Okeru Idenshi Ijo no Haaku ni Motozuita Gan no Hontai Kaimei to Sono Rinsho Oyo ni Kansuru Kenkyu' HEISEI 16 NENDO - 18 NENDO SOGO KENKYU HOKOKUSHO March 2007, pages 1 - 15, XP008138500

## Description

### Technical Field:

The present invention relates to a method for measuring a risk of acquiring liver cancer caused by a hepatitis virus or another cause. More specifically, the present invention relates to a method, a gene marker, and a diagnostic kit for determining the presence or absence of a genetic factor of liver cancer by detecting the presence or absence of methylation of a gene that affects a risk of liver cancer in a sample containing human genome DNA, such as a blood cell component or a plasma component collected from a subject. The invention also relates to a method for selecting a liver cancer therapeutic agent candidate.

### Background Art:

Liver cancer is a name referring to a malignant tumor occurring in the liver and can be roughly classified from its metastasis into a hepatocellular carcinoma, which primarily occurs in the liver cells, and metastatic liver cancer, which originates from an organ other than the liver and is transferred to the liver. A report of the Statistics Bureau in 1996 on the mortality from cancer shows that about 10000 patients die from liver cancer every year in Japan and that the mortality from liver cancer is 21.4% (1996), which is a major causative disease following stomach cancer, and, in 40s and 50s, the mortality from liver cancer is higher than that from stomach cancer.

Also, it has been recently reported that the number of deaths from liver cancer is 34000, which is about 11% of the total number of deaths from cancer. Ninety percent or more of the liver cancer patients are infected with hepatitis viruses, and it is also said that the number of viral hepatitis patients having a risk of developing liver cancer in Japan is three million. Hepatocellular carcinoma constitutes 95% of primary liver cancer, and the number of deaths therefrom reaches approximately 70% of all deaths from liver diseases. The causes of hepatocellular carcinoma are chronic hepatitis and liver cirrhosis due to hepatitis B and C, and the number of hepatocellular carcinoma cases is increasing each year. In about 70% of hepatitis C, the disease gradually progresses thereof and proceeds to liver cirrhosis and then liver cancer. It is known that approximately 80% of liver cancer cases are caused by hepatitis C.

Liver cancer is treated by resection of the liver, local treatment, hepatic artery therapeutic embolization, or radiotherapy. The resection of the liver is effective, provided that the liver function can endure the operation, and is most frequently employed. In the local treatment, a tumor is decreased in size by placing, for example, a needle into the tumor through the skin and injecting, for example, alcohol or is locally burned out by with a microwave or a radio wave. The hepatic artery therapeutic embolization kills cancer cells by occluding the hepatic artery to disconnect the cancer cells from their nutrient resource. Furthermore, the radiotherapy kills cancer cells by-irradiating the focus as a target with a radiation beam. However, at present, the surgical operation is the most effective means.

It is highly possible to increase the survival rate of liver cancer patients by treatment at an early stage. In the case of hepatocellular carcinoma, it is reported that the five-year survival rate of early-stage hepatocellular carcinoma patients is 45%, whereas the five-year survival rate of non-early-stage hepatocellular carcinoma patients is 11%. In addition, the five-year survival rate of patients in clinical disease stage I is 91%, and those in stages II and III are 12% and 0%, respectively. Accordingly, early detection of liver cancer is important, and it has been desired to develop, for example, a diagnostic agent that can conveniently and highly accurately diagnose liver cancer. In addition, the risk of recurrence after operation of liver cancer is high, and diagnosis of the recurrence is also necessary.

At present, AST (GOT), ALT (GPT), and γ-GTP (γ-glutamine transpeptidase) are widely used as indicators of liver dysfunction. The tests are used for diagnosing liver dysfunction, but diagnosis of inflammation, fibrillation, and progress to cancer further requires virus testing. Furthermore, liver cancer is diagnosed, for example, by diagnostic imaging or using a tumor marker. As the diagnostic imaging that can diagnose the site progressing to liver cirrhosis or liver cancer, an abdominal ultrasound test, CT, or MRI is employed. The CT test and the ultrasound test can detect early cancer even if its size is 10 mm or less and exhibit high accuracy. However, the tests require insurance approval, a technical expert level, and highly equipped facilities, which are disadvantageous for frequently testing and for enabling any medical institution to conduct the tests. Also, as the markers of hepatocellular carcinoma, for example, α-fetoprotein (AFP) and protein induced by vitamin K absence of antagonist (PIVKA-2; des-gamma-carboxyprothrombin) are known.

However, AFP and PIVKA-2 have a disadvantage that they have a low positive rate of about 30 to 40%. A subject with an AFP level of 20 ng/mL or more is determined to have hepatocellular carcinoma. However, since AFP also increases in non-liver cancer patients, such as chronic hepatitis and active liver cirrhosis, discrimination among mild and moderate cases is difficult. Therefore, an AFP-L3 fraction, which is a tumor marker exhibiting higher specificity for hepatocellular carcinoma, is used in some cases. On the other hand, PIVKA-2 is a tumor marker having high specificity for hepatocellular carcinoma and rarely increases in other diseases. However, an increased level thereof is observed in, for example, alcoholic liver injury, during drug administration (for example, Warfarin or an antitubercular drug), and during vitamin shortage, despite not having hepatocellular carcinoma.

In the actual diagnosis at present, liver cancer is diagnosed using combination of AFP, AFP-L3 fraction, and PIVKA-2. Diagnosis of liver cancer using a blood marker is required to have an improved diagnostic yield for liver cancer. That is, an effective diagnostic marker having increased specificity and sensitivity is necessary.

Accordingly, a new method is desired to be developed, and, for example, a candidate thereof is the use of aberrant methylation of P16 gene, which is a cancer-related gene, as an indicator (Non-Patent Literature 1). Furthermore, proposed is a diagnostic method for determining a subject having severe hepatitis C progressing to liver cancer by measuring the GPC3 level in a sample of a hepatitis C patient (Patent Literature 1).
Non-Patent Literature 1: Wong IHN, et al., Detection of aberrant p16 methylation in the plasma and serum of liver cancer patients, Cancer Res., 59, 71-73, 1999.
Patent Literature i: JP-A-2007-192557, Method for monitoring hepatitis C patient for progression to severe liver disease and liver cancer diagnostic method.

### Summary of Invention:

### Technical Problem:

However, conventional liver cancer markers or AFP and PIVKA-2 have disadvantages such that they are low in reactivity to early-stage cancer, that PIVKA-2 requires diagnostic equipment worth several million yen and needs time and effort because of combination with two or three types of other tumor markers, and that the type of tumor cannot be determined. Accordingly, an object to be solved by the present invention is to provide a convenient gene marker that has high reactivity also to early-stage cancer and high sensitivity for liver cancer and to provide a method for determining a risk of recurrence after resection of liver cancer. Furthermore, it is an object of the present invention to provide a method for searching a drug candidate that targets a methylated liver cancer marker gene.

### Solution to Problem:

The present inventors have conducted intensive studies to solve the above-mentioned problems. As a result, the inventors have found the fact that methylated human SALL3c gene, human ECEL1 gene, human FOXC1 gene, human NRG3 gene, and human KCNIP2 gene, and methylated gene regions represented by SEQ ID NO: 6 (NT_037622.5, 1393863 to 1395B63) and SEQ ID NO: 7 (NT_022135.15, 7774305 to 7776805) are liver cancer susceptible genes or gene regions. That is, it has been found that liver cancer can be detected by the presence or absence of methylation of these genes or gene regions or the frequency of the methylation. The present invention has proved that liver cancer can be detected using, as indicators, methylation of these genes or gene regions or a high frequency of the methylation, and has been thus accomplished. Furthermore, since a drug candidate that suppresses or prevents methylation of any of these genes has high possibility of becoming a liver cancer therapeutic drug, the present invention is also effective as a method for searching a drug candidate.

That is, the present invention provided the followings:

A method for detecting liver cancer as specified in the claims, in particular in claims 1 and 2.

The use of a polynucleotide for in-vitro diagnosis of liver cancer, as specified in the claims, in particular in claims 3, 6 and 7.

The use of a kit for in-vitro diagnosis of liver cancer as specified in the claims, in particular in claims 4 and 6 to 8.

The use of a microarray for in-vitro diagnosis of liver cancer as specified in the claims, in particular in claims 5 and 6 to 8.

A set of primers as specified in the claims, in particular in claim 9, wherein the SALL3c gene is an alternate splice form of SALL3 gene and has been newly found by the present inventors, who belong to Japanese Foundation For Cancer Research.

A method for detecting a risk of recurrence after operation of liver cancer as specified in the claims, in particular in claims 10 and 11.

The use of a polynucleotide for in-vitro diagnosis of the risk of recurrence after operation of liver cancer as specified in the claims, in particular in claims 12 to 16.

The use of a kit for in-vitro diagnosis of the risk of recurrence after operation of liver cancer as specified in the claims, in particular in Claims 13 and 15.

The use of a microarray for in-vitro diagnosis of the risk of recurrence after operation of liver cancer as specified in the claims, in particular in claims 14 and 15.

### Advantageous Effects of Invention:

According to the present invention, provided is a new detecting method that can detect liver cancer at low cost, in particular, for example, early-stage liver cancer and a risk of recurrence after operation of liver cancer and that is very convenient and highly reliable. Early detection of liver cancer is a most effective method for decreasing the death rate from cancer, and a diagnostic marker and a method for detection at an early stage are desired. Furthermore, an oligonucleotide, a microarray, and a diagnostic kit that are used for the detecting method can be also provided. In addition, it is possible to search a drug candidate that suppresses or inhibits the methylation by using the methylation of the genes as an indicator.

### Description of Embodiments:

The present invention will be described in detail below. Note that embodiments are not limited to the followings.

The present invention provides a method for detecting liver cancer, in particular, early-stage liver cancer and recurrence of cancer after operation. The method is based on the finding that methylation of human SALL3c gene, human ECEL1 gene, human FOXC1 gene, human NRG3 gene, human KCNIP2 gene, or a gene represented by SEQ ID NO: 6 (NT_037622.5, 1393863 to 1395863) or SEQ ID NO: 7 (NT_022135.15, 7774305 to 7776805) and a high frequency of the methylation are indicators highly specific to liver cancer, and focuses on the methylation of these genes and the methylated genes or gene regions.

The method for detecting liver cancer by measuring methylation of a specific gene according to the present invention can be performed as follows: First, in the detecting method of liver cancer in the present invention, a serum, plasma, or blood cell component is collected from a subject. Examples of the sample to be tested include interstitial fluid, extravascular fluid, cerebrospinal fluid, synovial fluid, saliva, and liver tissue. A sample after operation is preferably collected from third day to seventh day after the operation. The tissue sample is, for example, tissue extracted by operation or with an endoscope or part of the tissue, a biopsy sample, or organ-washing liquid. Furthermore, the method for extracting DNA from these samples is well known in the field of the art, and, for example, extraction with phenol/chloroform can be employed. Alternatively, a commercially available DNA extraction reagent may be used. In such a case, a commercially available genomic DNA extraction kit and apparatus, such as GFX Genomic Blood DNA Purification Kit, may be used. Furthermore, a specific gene may be directly detected by, for example, PCR without through treatment for extracting DNA from a sample. In addition, in the detecting method of the present invention, only several milliliters of blood or about 10 mg of tissue in resection of cancer is sufficient as a sample. Thus, the method is a significantly less invasive and therefore imposes a less burden on patients or subjects.

The term "methylation of DNA" refers to that a cytosine (C), a base, of the DNA is methylated (addition of CH₃). The methylation is observed, in many cases, at a CpG site where C is situated next to G, and approximately 80% of CpG sites are methylated. A region on a genome where the density of CpG sites is high is called a CpG island and is thought to be involved in, for example, regulation of gene expression, cancer, and imprinting. In general, cytosines in the CpG islands are rarely methylated, and the CpG sites outside the CpG islands are methylated. In addition, a region of DNA in which a methylated CpG site is generated on only one DNA strand during DNA replication is returned to a state that both DNA strands are methylated by a function of methyltransferase. Therefore, methylated CpG sites and unmethylated sites are conserved even after the replication of DNA, and it is known to function as a marker on a genome. As biological meaning of the methylation, in particular, when a CpG island is present in the 5' region of a gene, the methylation functions as an expression switch of the gene. Usually, the CpG island in the 5' region of a gene is not methylated so that the gene can be expressed. For example, in cancer, the CpG island in the 5' region of a tumor suppressor gene, which is usually unmethylated, is aberrantly methylated to suppress the expression, which has been recently recognized as an important oncogenic mechanism.

The term "nucleotide" refers to a nucleic acid. A polynucleotide is that nucleotides are joined to one another, includes DNA and RNA, and is the same meaning as a so-called primer, probe, or oligomer fragment.

In order to measure a degree or frequency of the methylation of DNA, for example, COBRA (combined bisulfite restriction analysis) or bisulfite sequencing can be used. These methods are based on a principle that unmethylated cytosine is converted to uracil, whereas methylated cytosine is not converted to uracil, by treating DNA with an unmethylated cytosine modifying reagent such as bisulfite. As technique for analyzing semi-quantitatively and with high sensitivity the methylation of DNA, Methylight is known. In the Methylight, methylation is detected by real-time PCR using combination of a primer recognizing a methylation specific sequence and a TaqMan probe. Furthermore, another example is a method using a restriction enzyme (such as Dnp1) that specifically cleaves a methylated gene sequence.

### Gene sequence information:

Information about genes that can be used in the method of the present invention is cited in the following Table 1 (SEQ ID NOs: 1 to 7).

Table 1 shows SEQ ID NOs., gene names, and Accession Nos. of genes that can be used in the present invention.

Any of the gene sequence information can be obtained using the Accession Nos. that can be confirmed in the website (http://www.ncbi.nlm.nih.gov/) of the National Biotechnology Information Center (NCBI). SALL3C, which does not have an Accession No., has been identified by the present inventors belonging to Japanese Foundation For Cancer Research as a new alternate splice form of SALL3 (NM_171999) and is planed to be registered to a gene bank (NCBI). The gene sequence of SALL3C is shown as SEQ ID NO: 1. In the genes not named, the functions thereof are unclear whereas the gene sequence information is known. These genes not named are shown by the gene region positions that are used in the present invention.

**[Table 1].**

| SEQ ID NO: | Gene Name (Gene Region Position) | Accession No. |
|---|---|---|
| SEQ ID NO: 1 | sal-like 3C (SALL3C) | SEQ ID NO: 1 |
| SEQ ID NO: 2 | Endothelin converting enzyme-like I (ECEL1) | NM_004826 |
| SEQ ID NO: 3 | Forkhead box (FOXC1) | NM_001453 |
| SEQ ID NO: 4 | neuregulin 3 (NRG3) | NM_001010848 |
| SEQ ID NO: 5 | Kv channel interacting protein 2 (KCNIP2) | NM_014591 |
| SEQ ID NO: 6 | 1393863 to 1395863 | NT_037622.5 |
| SEQ ID NO: 7 | 7774305 to 7776805 | NT_022135.15 |

### Detecting method of methylation in gene according to the present invention:

A detecting method of methylation in a specific gene used in the detecting method of the present invention will be exemplarily shown below.

As technique for analyzing semi-quantitatively and with high sensitivity the methylation of DNA, Methylight and bisulfite sequencing are known. In the bisulfite sequencing, a single-chain DNA is treated with bisulfite (sodium hydrogen sulfite) to cause sulfonation and hydroamination. Subsequently, desulfonation is performed to convert cytosine to uracil. On the other hand, since the sulfonation rate of methylated cytosine is very slow, the methylated cytosine is not converted to uracil, but unmethylated cytosine is substituted by thymine (Frommer M, et al., Proc. Natl. Acad. Sci. USA, 891827-1831, (1992), Clark S J, et al., Nucleic Acids Res., 22, 2990 (1994)). The methylation status can be analyzed by utilizing the difference (C and T) in sequences. There are a number of methods utilizing bisulfite treatment for more specific experiments, such as bisulfite sequencing and combined bisulfite restriction analysis (COBRA), and a method can be selected from these methods so as to be suitable for the analysis purpose.

The DNA used for the detection of methylation is treated as follows: First, DNA is extracted from blood cells or serum using DNeasy Blood & Tissue kits (Qiagen). Subsequently, the DNA sample extracted from blood cells or serum is treated with bisulfite. A small amount of serum DNA or 500 ng of DNA extracted from blood cells or tissue is denatured by treatment with 0.2 M NaCl at 37°C for 10 minutes. Then, sodium hydrogen sulfite in a final concentration of 3 M and hydroquinone in a final concentration of 0.5 mM are added thereto, followed by reaction for 16 hours. The reaction solution is desalted using Wizard DNA purification kit. The bisulfite treatment is performed in 0.3 M NaOH for 15 minutes and then is terminated. The modified DNA is precipitated with ethanol and then washed with 70% ethanol. The modified DNA is dissolved in 20 µL of distilled water. Alternatively, Epi TectBisulfite Kit (Qiagen) is used.

Then, primers for specifically detecting each of the methylated genes are prepared for detecting the presence or absence of methylation in the genes or gene regions shown in Table 1 or a frequency of the methylation according to the present invention. Table 2 shows the sequences (from 5' to 3') of sets of primers that can detect the respective genes used in Examples of the present invention. Then, nested PCR is performed using primers F1 and R1 in the first PCR and primers F2 and R2 in the second PCR. The nested PCR is a method for performing two-step PCR using external primers and internal primers. The first PCR products from an intended region are used as templates, and both primers are designed so as to recognize inner sides than the positions of the primers used first. The primer sequences are shown in Table 2, but are not limited thereto as long as they can specifically detect or identify the methylation of the genes. In the present invention, the marker for diagnosis of acquiring liver cancer may be primers that can amplify these specific genes. In order to amplify a gene sample by PCR, DNA polymerase having high fidelity is preferred. The primers are designed and synthesized using part of the gene sequence information shown in Table 2 in such a manner that the methylated specific gene as a target can be amplified. After the completion of the amplification reaction, the amplified products are detected for the presence or absence of methylation or a frequency of the methylation. In the present invention, the full length of each above-mentioned gene is not necessarily measured or detected, and a part of the gene may be measured or detected provided that each gene can be specified.

**[Table 2]**

| Gene SEQ ID NO: Gene Name, etc. | Primer Sequence |
|---|---|
| SEQ ID NO: 1 | F1: gttcgggttggtcgtttatcgttc (SEQ ID NO: 8) |
| SALL3C | R1: cccacacactcgacccctaacg (SEQ ID NO: 9) |
| | F2: agacgtattggggcgaggggc (SEQ ID NO: 10) |
| | R2: ctccccgcgatcacacgcacg (SEQ ID NO:11) |
| SEQ ID NO: 2 | F1: tttcgcggagacgttaatttagttc (SEQ ID NO: 12) |
| ECEL1 | R1: aaacgcaaaacgtatacaacgccg (SEQ ID NO: 13) |
| | F2: gagggttgggaaattgcggttttc (SEQ ID NO: 14) |
| | R2: ctcctcgcgctacgtcatccg (SEQ ID NO: 15) |
| SEQ ID NO: 3 | F1: gcgggtcggtattagttcggtc (SEQ ID NO: 16) |
| FOXC1 | R1: ctacgacgtataaaacccgtaaacg (SEQ ID NO: 17) |
| | F2: ggggttatgtaggcgcgttatttc (SEQ ID NO: 18) |
| | R2: tacgaatacacgctcataaaaaccg (SEQ ID NO: 19) |
| SEQ ID NO: 4. | F1: gggattcggcgcgtaggaggc (SEQ ID NO: 20) |
| NRG3 | R1: caacgtaactcccgaaaaaactcg (SEQ ID NO: 21) |
| | F2: gtcgttttttgatcgatcggaggc (SEQ ID NO: 22) |
| | R2: aaaccgcgacaaaaacataaaaccg (SEQ ID NO: 23) |
| SEQ ID NO: 5 | F1: ttcgtttttcggttttattcgatgtc (SEQ ID NO: 24) |
| KCNIP2 | R1: actaaacgaatctaaattaatccccg (SEQ ID NO: 25) |
| | F2: gatggttattttcgaggtttattagc (SEQ ID NO: 26) |
| | R2: cctccctttctaaaacgaaaatacg (SEQ ID NO: 27) |
| SEQ ID NO: 6 | F1: agtataatatatcgcgtttataaattatc (SEQ ID NO: 28) |
| NT_037622.5 | R1: aacgacgcgacttatcgaacacg (SEQ ID NO: 29) |
| 1393863 to 1395863 | F2: gcgttttttatttaatgtaaatggagc (SEQ ID NO: 30) |
| | R2: taatcgcgacatcaaccatcgacg (SEQ ID NO: 31) |
| SEQ ID NO: 7 | F1: gagagtacgttagttttggagattc (SEQ ID NO: 32) |
| NT_022135.15 | R1: cacgtactttccctccttaactcg (SEQ ID NO: 33) |
| 7774305 to 7776805 | F2: ttagtattgcgaatagcgttagtatc (SEQ ID NO: 34) |
| | E2: aataaatactaacttaatcgaaataaacg (SEQ ID NO: 35) |

The gene amplification (PCR) in Examples of the present invention is performed as follows: Since a gene sample to be tested includes a large number of templates that cannot be amplified, Tth polymerase, which is a powerful tool, is used, and 2 µL of bisulfite-treated DNA is used as a template. The PCR reaction solution in the first PCR is prepared as shown in Table 3 using GeneAmp XL PCR Kit available from Applied Biosystems.

**[Table 3]**

| | |
|---|---|
| Template DNA | 2 µL |
| 3.3X XL buffer II | 15 µL |
| 25 mM Mg(OAc)₂ | 2.2 µL |
| 10 mM dNTP mix | 1 µL |
| primer A | 1 µL |
| primer B | 1 µL |
| rTth polymerase | 0.5 µL |
| Dw | 27.3 µL |

In the first step of PCR, a reaction cycle of denature at 94 °C for 30 seconds, annealing at 53 to 54°C for 30 seconds, and extension at 68 °C for 3 minutes is repeated 40 times. Then, the PCR reaction solution for the second step of PCR is prepared as shown in Table 4 using GeneAmp XL PCR Kit available from Applied Biosystems. The PCR product in the first step is diluted with distilled water to 1/500 (in the case of serum DNA) or 1/1000 (in the case of blood cell DNA), and 1 µL of the diluted PCR product is used as the template DNA. In the second step of PCR, a reaction cycle of denature at 94 °C for 30 seconds, annealing at 53 to 54 °C for 30 seconds, and extension at 68°C for 3 minutes is repeated 40 times.

**[Table 4]**

| | |
|---|---|
| Template DNA | 1 µL |
| 3.3X XL buffer II | 15 µL |
| 25 mM Mg(OAc)₂ | 2.2 µL |
| 10 mM dNTP mix | 1 µL |
| primer A | 1 µL |
| primer B | 1 µL |
| rTth polymerase | 0.5 µL |
| Dw | 28.3 µL |

### Kit for diagnosis of acquiring liver cancer:

In the present invention, the kit for diagnosis of acquiring liver cancer includes, in addition to the primers that can amplify each of the genes shown above, one or more components that are necessary for conducting the present invention. For example, the kit of the present invention can include a component for preserving or supplying an enzyme and a reaction component necessary for conducting the PCR. Examples of such components include, but not limited thereto, oligonucleotides of the present invention, enzyme buffer, dNTP, control reagents (such as target oligonucleotides for tissue samples and positive and negative controls), reagents for labeling or detection, a solid-phase support, and a manual.

### Method for selecting liver cancer therapeutic agent candidate:

The method for selecting a liver cancer therapeutic agent candidate includes the steps of culturing mammalian cells in the presence and absence of a test compound, measuring a frequency of methylation of human SALL3c gene, human ECEL1 gene, human FOXC1 gene, human NRG3 gene, human KCNIP2 gene, SEQ ID NO: 6 (NT_037622.5, 1393863 to 1395863), or SEQ ID NO: 7 (NT_022135.15, 7774305 to 7776805) in each cell, and selecting a test compound having an effect of suppressing the methylation as a liver cancer therapeutic agent candidate. The present invention is based on the finding that since the methylation of the genes and the gene regions is associated with liver cancer, a compound that can suppress or inhibit the methylation is identified as a liver cancer therapeutic agent candidate. Herein, cultured mammalian cells are used because methylation does not occur in yeast and Escherichia coli cells, or the physiological meaning of methylation differs from that of the present invention even if it occurs.

The present invention will be described more specifically with reference to Examples below, but is not limited thereto.

### Example 1:

### Detection rate in each stage of liver cancer of each.gene marker:

Table 5 shows the results of a test of each gene marker for characteristics that can detect early-stage cancer in patients having liver cancer that is classified into well-differentiated (early-stage) cancer and moderately- or poorly-differentiated (advanced) cancer. All the patients are scheduled to be operated for resecting the liver cancer irrespective of sex. In addition, the classification to well-differentiation or moderately- or poorly-differentiation was accordance with the findings of medical doctors in charge of operation of the liver cancer. In order to evaluate the detection rates of liver cancer according to the present invention, detection rates of AFP and PIVKA-2, which are liver cancer markers conventionally used, were similarly investigated.

As shown in Table 5, though the AFP marker, which is conventionally used, showed low detection rates at every stages of liver cancer, the PIVKA-2 marker showed a high detection rate of 70% in well-differentiated cancer and a lower detection rate than the above of 44% in moderately- and poorly-differentiated cancer. The methylated SALL3C, ECEL1, and NT_037622.5 (1393863 to 1395863) of the present invention showed high detection rates of 80%, 70%, and 80%, respectively, in well-differentiated cancer, which are higher than the detection rate of the PIVKA-2 marker and show their effectiveness. Here, the calculated detection rate is a ratio of the number of patients that reacted to a gene marker (a group in which methylated gene is detected) to the number of patients having well-differentiated cancer or moderately- or poorly-differentiated cancer. Furthermore, it is also possible to detect moderately- or poorly-differentiated cancer in consideration of-that clinical research showing "any one of the markers is positive before operation and changes to negative after the operation" and that healthy individuals show negative in any of the genes in Example 3.

**[Table 5]**

| SEQ ID NO:, Accession No., Gene Name (Gene Region Position) | Well-differentiated Cancer | Moderately- or Poorly-differentiated Cancer |
|---|---|---|
| SEQ ID NO: 1 SALL3C | 8 subjects (80%) | 24 subjects (53%) |
| SEQ ID NO: 2, NM_004826 ECEL1 | 7 subjects (70%) | 17 subjects (38%) |
| SEQ ID NO: 3, NM_001453 FOXC1 | 5 subjects (50%) | 10 subjects (22%) |
| SEQ ID NO: 4, NM_001010848 NRG3 | 4 subjects (40%) | 8 subjects (18%) |
| SEQ ID NO: 5, NM_014591 KCNIP2 | 2 subjects (20%) | 5 subjects (11%) |
| SEQ ID NO: 6, NT_037622.5 1393863 to 1395863 | 8 subjects (80%) | 28 subjects (62%) |
| SEQ ID NO: 7, NT_022135.15 7774305 to 7776805 | 4 subjects (40%) | 2 subjects (4%) |
| AFP | 2 subjects (20%) | 11 subjects (24%) |
| PIVKA II | 7 subjects (70%) | 20 subjects (44%) |

### Example 2

### Detection rate in vascular invasion of liver cancer of each gene marker:

The vascular invasion of liver cancer means that cancer cells invade the circumference of the portal vein, the hepatic vein, the hepatic artery, and the bile duct or into these vascular systems, and includes macroscopic vascular invasion that can be diagnosed by image inspection or visual inspection of a resected specimen and microscopic vascular invasion that can be first diagnosed under a microscope. The microscopic vascular invasion is known as one of the most powerful prognostic factors against recurrence after treatment, but it is difficult for the current technology to diagnose before operation whether or not the microscopic vascular invasion is present. The results shown in Table 6 show the AFP marker and the PIVKA-2 marker, which are conventionally used, cannot determine the presence or absence of vascular invasion of liver cancer. On the other hand, the methylated ECEL1 and NT_037622.5 (1393863 to 1395863) of the present invention exhibited detection rates of 55% and 85%, respectively, against liver cancer having vascular invasion and, at the same time, exhibited detection rates of 37% and 54%, respectively, against liver cancer not having vascular invasion. This means that the use of these two gene markers makes it possible to anticipate whether or not the liver cancer has vascular invasion. Here, the calculated detection rate is a ratio of the number of patients that reacted to a gene marker (a group in which methylated gene is detected) to the number of cancer patients having vascular invasion or the cancer patients not having vascular invasion. On the other hand, since the detection rates of methylated SALL3c were 45% in liver cancer having vascular invasion and 66% in liver cancer not having vascular invasion, liver cancer not having vascular invasion can be detected.

**[Table 6]**

| SEQ ID NO:, Accession No., Gene Name (Gene Region Position) | With vascular invasion | Without vascular invasion |
|---|---|---|
| SEQ ID NO: 1 SALL3C | 9 subjects (45%) | 23 subjects (66%) |
| SEQ ID NO: 2, NM_004826 ECEL1 | 11 subjects (55%) | 13 subjects (37%) |
| SEQ ID NO: 3, NM_001453 FOXC1 | 7 subjects (35%) | 8 subjects (23%) |
| SEQ ID NO: 4, NM_001010848 NRG3 | 4 subjects (20%) | 8 subjects (23%) |
| SEQ ID NO: 5, NM_014591 KCNIP2 | 2 subjects (10%) | 5 subjects (14%) |
| SEQ ID NO: 6, NT_037622.5 1393863 to 1395863 | 17 subjects (85%) | 19 subjects (54%) |
| SEQ ID NO: 7, NT_022135.15 7774305 to 7776805 | 4 subjects (20%) | 8 subjects (23%) |
| AFP | 6 subjects (30%) | 7 subjects (20%) |
| PIVKA II | 8 subjects (40%) | 19 subjects (54%) |

### Example 3

### Reaction of each gene marker before and after resection operation of liver cancer:

In the present invention, in particular, the methylated SALL3C, ECEL1, and NT_037622.5 (1393863 to 1395863), which exhibit high liver cancer detection rates, were inspected before and after resection operation for whether or not the gene marker was detected. Furthermore, patient samples (specimens) were evaluated for plasma and blood cells. The results are shown by (-/-) meaning that the marker was not detected before and after operation, (+/+) meaning that the marker was detected before and after operation, (+/-) meaning that the marker was detected before operation but was not detected after the operation, and (-/+) meaning that the marker was not detected before operation but was detected after the operation. In working assumption, a disease marker that is detected before operation and not detected after operation, (+/-), is assumed to be effective. On the other hand, a marker showing (-/+) is assumed that the detection rate is low, and a marker showing (+/+), which is detected before and after operation despite of enucleation of liver cancer, is also assumed that the detection rate is low. However, since these disease markers may not be effective depending on, for example, the constitution of a patient in some cases, it is assumed that a high detection rate can be obtained compared to (-/+) and (+/+).

Then, the detection rates and non-detection rates of each gene marker before and after operation were calculated from the results of actual tests (Table 7) . In Table 7, (-) means that a methylated gene was not detected (negative), and (+) means that a methylated gene was detected (positive) . The results show that the methylated SALL3C, ECEL1, and NT_037622.5 (1393863 to 1395863) extracted from blood cells exhibited high detection rates of 56%, 42%, and 64%, respectively, as those that are detected before operation and not detected after operation (+/-) . On the other hand, these gene markers exhibited low detection rates of 20%, 36%, and 18%, respectively, as those that are also detected after operation (+/+), responding to cancer-enucleating operation. Furthermore, all of these gene markers exhibited low detection rates of 2% as those that are not detected before operation and detected after operation, but these gene markers as those that are not detected both before and after operation (-/-) exhibited low detection rates of 22%, 20%, and 16%, respectively. This shows that in liver cancer patients, the detection rates of the methylated SALL3C, ECEL1, and NT_037622.5 (1393863 to 1395863) gene markers before and after operation are logical in patent groups in which these gene markers are detected, and the reliability thereof is high, though there is a group, at a certain ratio, in which the gene markers are not detected because of, for example, genetic constitution. Furthermore, the average (average of three gene markers) of the patient group in which the gene markers are not detected is 19%, but the average of the patient group in which the gene markers are detected is high, 54%, which shows that the gene markers are highly effective. In addition, DNA extracted from blood cells exhibited higher specificity and sensitivity as a test sample of a patient.

In each Example, sera and blood cells of five healthy individuals (34-year-old male, 34-year-old female, 50-year-old male, 63-year-old make, and 64-year-old female) who are known to be HBsAg (antigen of hepatitis B) negative and HCV Ab (antibody of hepatitis C) negative were similarly subjected to PCR and were thereby confirmed that methylation of the seven genes and gene regions according to the present invention was negative.

**[Table 7]**

| Gene Marker | Sample | -/- | +/- | +/+ | -/+ |
|---|---|---|---|---|---|
| SALL3C | plasma | 50 subjects (91%) | 4 subjects (7%) | 0 subject (0%) | 1 subject (2%) |
| SEQ ID NO:1 | blood cell | 12 subjects (22%) | 31 subjects (56%) | 11 subjects (20%) | 1 subject (2%) |
| ECEL1 | plasma | 46 subjects (84%) | 9 subjects (16%) | 0 subject (0%) | 0 subject (0%) |
| SEQ ID NO: 2 | blood cell | 11 subjects (20%) | 23 subjects (42%) | 20 subjects (36%) | 1 subject (2%) |
| NT_037622.5 | plasma | 50 subjects (91%) | 3 subjects (5%) | 0 subject (0%) | 2 subjects (4%) |
| 1393863 to 1395863 SEQ ID NO: 6 | blood cell | 9 subjects (16%) | 35 subjects (64%) | 10 subjects (18%) | 1 subject (2%) |

## Claims

1. A method for detecting liver cancer by detecting in a sample the presence of methylation of any one or more genes or gene regions selected from the group consisting of human SALL3c gene, human ECEL1 gene, human FOXC1 gene, human NRG3 gene and human KCNIP2 gene, a gene region represented by SEQ ID NO: 6 (NT_037622.5, 1393861 to 1395960) and a gene region represented by SEQ ID NO: 7 (NT_022135.15, 7774305 to 7776805) that is detectable using a set of primers of SEQ ID NO: 8 and SEQ ID NO: 9 for human SALL3c gene, a set of primers of SEQ ID NO:12 and SEQ ID NO:13 for human ECEL1 gene, a set of primers of SEQ ID NO:16 and SEQ ID NO:17 for human FOXC1 gene, a set of primers of SEQ ID NO: 20 and SEQ ID NO: 21 for human NRG3 gene, a set of primers of SEQ ID NO: 24 and SEQ ID NO: 25 for human KCNIP2 gene, a set of primers of SEQ ID NO: 28 and SEQ ID NO: 29 for the gene region represented by SEQ ID NO: 6, and a set of primers of SEQ ID NO: 32 and SEQ ID NO: 33 for the gene region represented by SEQ ID NO: 7, wherein the sample is selected from the group consisting of blood and liver tissue of a subject.

2. The method according to Claim 1, wherein the methylation is detected or identified by a set of primers selected from the group consisting of: a set of primers of SEQ ID NO: 8 and SEQ ID NO: 9, and/or a set of primers of SEQ ID NO:10 and SEQ ID NO:11 for human SALL3c gene; a set of primers of SEQ ID NO:12 and SEQ ID NO:13, and/or a set of primers of SEQ ID NO:14 and SEQ ID NO:15 for human ECEL1 gene; a set of primers of SEQ ID NO:16 and SEQ ID NO:17, and/or a set of primers of SEQ ID NO:18 and SEQ ID NO:19 for human FOXC1 gene; a set of primers of SEQ ID NO: 20 and SEQ ID NO: 21, and/or a set of primers of SEQ ID NO: 22 and SEQ ID NO: 23 for human NRG3 gene; a set of primers of SEQ ID NO: 24 and SEQ ID NO: 25, and/or a set of primers of SEQ ID NO: 26 and SEQ ID NO: 27 for human KCNIP2 gene; a set of primers of SEQ ID NO: 28 and SEQ ID NO: 29, and/or a set of primers of SEQ ID NO: 30 and SEQ ID NO: 31 for the gene region represented by SEQ ID NO: 6; a set of primers of SEQ ID NO: 32 and SEQ ID NO: 33, and/or a set of primers of SEQ ID NO: 34 and SEQ ID NO: 35 for the gene region represented by SEQ ID NO: 7.

3. Use of a polynucleotide for in-vitro diagnosis of liver cancer, **characterized in that** the polynucleotide can detect the methylation of at least one of the genes or gene regions selected from the group consisting of:
(1) human SALL3c gene,
(2) human ECEL1 gene,
(3) human FOXC1 gene,
(4) human NRG3 gene,
(5) human KCNIP2 gene,
(6) SEQ ID NO: 6 (NT_037622.5, 1393861 to 1395960), and
(7) SEQ ID NO: 7 (NT_022135.15, 7774305 to 7776805)
that is detectable using a set of primers of SEQ ID NO: 8 and SEQ ID NO: 9 for human SALL3c gene, a set of primers of SEQ ID NO:12 and SEQ ID NO: 13 for human ECEL1 gene, a set of primers of SEQ ID NO:16 and SEQ ID NO:17 for human FOXC1 gene, a set of primers of SEQ ID NO: 20 and SEQ ID NO: 21 for human NRG3 gene, a set of primers of SEQ ID NO: 24 and SEQ ID NO: 25 for human KCNIP2 gene, a set of primers of SEQ ID NO: 28 and SEQ ID NO: 29 for SEQ ID NO: 6, and a set of primers of SEQ ID NO: 32 and SEQ ID NO: 33 for SEQ ID NO: 7.

4. Use of a kit for in-vitro diagnosis of liver cancer, **characterized in that** the kit comprises one or more polynucleotides that can detect the methylation of at least one of the genes or gene regions selected from the group consisting of:
(1) human SALL3c gene,
(2) human ECEL1 gene,
(3) human FOXC1 gene,
(4) human NRG3 gene,
(5) human KCNIP2 gene,
(6) SEQ ID NO: 6 (NT_037622.5, 1393861 to 1395960), and
(7) SEQ ID NO: 7 (NT_022135.15, 7774305 to 7776805)
that is detectable using a set of primers of SEQ ID NO: 8 and SEQ ID NO: 9 for human SALL3c gene, a set of primers of SEQ ID NO:12 and SEQ ID NO:13 for human ECEL1 gene, a set of primers of SEQ ID NO:16 and SEQ ID NO: 17 for human FOXC1 gene, a set of primers of SEQ ID NO: 20 and SEQ ID NO: 21 for human NRG3 gene, a set of primers of SEQ ID NO: 24 and SEQ ID NO: 25 for human KCNIP2 gene, a set of primers of SEQ ID NO: 28 and SEQ ID NO: 29 for SEQ ID NO: 6, and a set of primers of SEQ ID NO: 32 and SEQ ID NO: 33 for SEQ ID: NO: 7.

5. Use of a microarray for in-vitro diagnosis of liver cancer, **characterized in that** the microarray comprises one or more polynucleotides that can detect the methylation of at least one of the genes or gene regions selected from the group consisting of:
(1) human SALL3c gene,
(2) human ECEL1 gene,
(3) human FOXC1 gene,
(4) human NRG3 gene,
(5) human KCNIP2 gene,
(6) SEQ ID NO: 6 (NT_037622.5,1393861 to 1395960), and
(7) SEQ ID NO: 7 (NT_022135.15, 7774305 to 7776805)
that is detectable using a set of primers of SEQ ID NO: 8 and SEQ ID NO: 9 for human SALL3c gene, a set of primers of SEQ ID NO:12 and SEQ ID NO:13 for human ECEL1 gene, a set of primers of SEQ ID NO: 16 and SEQ ID NO:17 for human FOXC1 gene, a set of primers of SEQ ID NO: 20 and SEQ ID NO: 21 for human NRG3 gene, a set of primers of SEQ ID NO: 24 and SEQ ID NO: 25 for human KCNIP2 gene, a set of primers of SEQ ID NO: 28 and SEQ ID NO: 29 for SEQ ID NO: 6, and a set of primers of SEQ ID NO: 32 and SEQ ID NO: 33 for SEQ ID NO: 7.

6. The use according to any one of Claims 3-5, wherein the polynucleotide is selected from the group consisting of: a set of primers of SEQ ID NO: 8 and SEQ ID NO: 9, and/or a set of primers of SEQ ID NO:10 and SEQ ID NO:11 for human SALL3c gene; a set of primers of SEQ ID NO:12 and SEQ ID NO:13, and/or a set of primers of SEQ ID NO:14 and SEQ ID NO:15 for human ECEL1 gene; a set of primers of SEQ ID NO:16 and SEQ ID NO:17, and/or a set of primers of SEQ ID NO:18 and SEQ ID NO:19 for human FOXC1 gene; a set of primers of SEQ ID NO: 20 and SEQ ID NO: 21, and/or a set of primers of SEQ ID NO: 22 and SEQ ID NO: 23 for human NRG3 gene; a set of primers of SEQ ID NO: 24 and SEQ ID NO: 25, and/or a set of primers of SEQ ID NO: 26 and SEQ ID NO: 27 for human KCNIP2 gene, a set of primers of SEQ ID NO: 28 and SEQ ID NO: 29, and/or a set of primers of SEQ ID NO: 30 and SEQ ID NO: 31 for the gene region represented by SEQ ID NO: 6; a set of primers of SEQ ID NO: 32 and SEQ ID NO: 33, and/or a set of primers of SEQ ID NO: 34 and SEQ ID NO: 35 for the gene region represented by SEQ ID NO: 7.

7. The use according to any one of Claims 3-6, wherein the gene or gene region is selected from the group consisting of :
(1) human SALL3c gene,
(2) human ECEL1 gene, and
(3) SEQ ID NO: 6 (NT_037622.5, 1393861 to 1395960).

8. Use of a polynucleotide according to Claim 4 or 5 for manufacturing a detection kit or microarray for detecting liver cancer.

9. A set of primers selected from the group consisting of: a set of primers of SEQ ID NO: 8 and SEQ ID NO: 9 for human SALL3c gene, a set of primers of SEQ ID NO:10 and SEQ ID NO:11 for human SALL3c gene, a set of primers of SEQ ID NO:12 and SEQ ID NO: 13 for human ECEL1 gene, a set of primers of SEQ ID NO:14 and SEQ ID NO: 15 for human ECEL1 gene, a set of primers of SEQ ID NO:16 and SEQ ID NO:17 for human FOXC1 gene, a set of primers of SEQ ID NO:18 and SEQ ID NO: 19 for human FOXC1 gene, a set of primers of SEQ ID NO: 20 and SEQ ID NO: 21 for human NRG3 gene, a set of primers of SEQ ID NO: 22 and SEQ ID NO: 23 for human NRG3 gene, a set of primers of SEQ ID NO: 24 and SEQ ID NO: 25 for human KCNIP2 gene, a set of primers of SEQ ID NO: 26 and SEQ ID NO: 27 for human KCNIP2 gene; a set of primers of SEQ ID NO: 28 and SEQ ID NO: 29 for the gene region represented by SEQ ID NO: 6, a set of primers of SEQ ID NO: 30 and SEQ ID NO: 31 for the gene region represented by SEQ ID NO: 6, a set of primers of SEQ ID NO: 32 and SEQ ID NO: 33 for the gene region represented by SEQ ID NO: 7, and a set of primers of SEQ ID NO: 34 and SEQ ID NO: 35 for the gene region represented by SEQ ID NO: 7.

10. A method for detecting a risk of recurrence after operation of liver cancer by detecting in a sample the presence of methylation of any one or more genes or gene regions selected from the group consisting of human SALL3c gene and human ECEL1 gene, and a gene region represented by SEQ ID NO: 6 (NT_037622.5, 1393861 to 1395960) that is detectable using a set of primers of SEQ ID NO: 8 and SEQ ID NO: 9 for human SALL3c gene, a set of primers of SEQ ID NO: 12 and SEQ ID NO:13 for human ECEL1 gene, and a set of primers of SEQ ID NO: 28 and SEQ ID NO: 29, wherein the sample is selected from the group consisting of blood and liver tissue of a subject.

11. The method according to Claim 10, wherein the methylation is detected or identified by a set of primers selected from the group consisting of: a set of primers of SEQ ID NO: 8 and SEQ ID NO: 9, and/or a set of primers of SEQ ID NO:10 and SEQ ID NO:11 for human SALL3c gene; a set of primers of SEQ ID NO:12 and SEQ ID NO:13, and/or a set of primers of SEQ ID NO: 14 and SEQ ID NO:15 for human ECEL1 gene; and a set of primers of SEQ ID NO: 28 and SEQ ID NO: 29, and/or a set of primers of SEQ ID NO: 30 and SEQ ID NO: 31 for the gene region represented by SEQ ID NO: 6.

12. Use of a polynucleotide for in-vitro diagnosis of the risk of recurrence after operation of liver cancer, **characterized in that** the polynucleotide can detect the methylation of at least one of the genes or gene regions selected from the group consisting of human SALL3c gene, human ECEL1 gene, and/or the methylation of the gene regions SEQ ID NO: 6 (NT_037622.5,1393861 to 1395960), that is detectable using a set of primers of SEQ ID NO: 8 and SEQ ID NO: 9 for human SALL3c gene, a set of primers of SEQ ID NO:12 and SEQ ID NO:13 for human ECEL1 gene, and a set of primers of SEQ ID NO: 28 and SEQ ID NO: 29 for the gene region represented by SEQ ID NO: 6.

13. Use of a kit for in-vitro diagnosis of the risk of recurrence after operation of liver cancer, **characterized in that** the kit comprises one or more polynucleotides that can detect the methylation of at least one of the genes or gene regions selected from the group consisting of human SALL3c gene, human ECEL1 gene, and/or the methylation of the gene regions SEQ ID NO: 6 (NT_037622.5, 1393861 to 1395960), that is detectable using a set of primers of SEQ ID NO: 8 and SEQ ID NO: 9 for human SALL3c gene, a set of primers of SEQ ID NO:12 and SEQ ID NO:13 for human ECEL1 gene, and a set of primers of SEQ ID NO: 28 and SEQ ID NO: 29 for the gene region represented by SEQ ID NO: 6.

14. Use of a microarray for in-vitro diagnosis of the risk of recurrence after operation of liver cancer, **characterized in that** the microarray comprises one or more polynucleotides that can detect the methylation of at least one of the genes or gene regions selected from the group consisting of human SALL3c gene, human ECEL1 gene, and/or the methylation of the gene regions SEQ ID NO: 6 (NT_037622.5, 1393861 to 1395960), that is detectable using a set of primers of SEQ ID NO: 8 and SEQ ID NO: 9 for human SALL3c gene, a set of primers of SEQ ID NO:12 and SEQ ID NO:13 for human ECEL1 gene, and a set of primers of SEQ ID NO: 28 and SEQ ID NO: 29 for the gene region represented by SEQ ID NO: 6.

15. The use according to any one of Claims 12-14, wherein the polynucleotide is selected from the group consisting of: a set of primers of SEQ ID NO: 8 and SEQ ID NO: 9, and/or a set of primers of SEQ ID NO:10 and SEQ ID NO: 11 for human SALL3c gene; a set of primers of SEQ ID NO:12 and SEQ ID NO:13, and/or a set of primers of SEQ ID NO:14 and SEQ ID NO:15 for human ECEL1 gene; and a set of primers of SEQ ID NO: 28 and SEQ ID NO: 29, and/or a set of primers of SEQ ID NO: 30 and SEQ ID NO: 31 for the gene region represented by SEQ ID NO: 6.

16. Use of a polynucleotide according to Claim 13 or 14 for manufacturing a detection kit or microarray for detecting the risk of recurrence after operation of liver cancer.

## Patentansprüche

1. Verfahren zum Nachweis von Leberkrebs, bei dem in einer Probe das Vorhandensein der Methylierung eines oder mehrerer Gene oder Genregionen nachgewiesen wird, das/die ausgewählt ist/sind aus der Gruppe bestehend aus: humanem SALI3c Gen, humanem ECEL1 Gen, humanem FOXC1 Gen, humanem NRG3 Gen und humanem KCNIP2 Gen, einer durch SEQ ID NO: 6 (NT_037622.5, 1393861 bis 1395960) repräsentierten Genregion und einer durch SEQ ID NO: 7 (NT_022135.15, 7774305 to 7776805) repräsentierten Genregion, das/die detektierbar ist unter Verwendung eines Primer-Sets aus SEQ ID NO: 8 und SEQ ID NO: 9 für humanes SALL3c Gen, eines Primer-Sets aus SEQ ID NO: 12 und SEQ ID NO: 13 für humanes ECEL1 Gen, eines Primer-Sets aus SEQ ID NO: 16 und SEQ ID NO: 17 für humanes FOXC1 Gen, eines Primer-Sets aus SEQ ID NO: 20 und SEQ ID NO: 21 für humanes NRG3 Gen, eines Primer-Sets aus SEQ ID NO: 24 und SEQ ID NO: 25 für humanes KCNIP2 Gene, eines Primer-Sets aus SEQ ID NO: 28 und SEQ ID NO: 29 für die durch SEQ ID NO: 6 repräsentierte Genregion und eines Primer-Sets aus SEQ ID NO: 32 und SEQ ID NO: 33 für die durch SEQ ID NO: 7 repräsentierte Genregion, wobei die Probe ausgewählt ist aus der Gruppe bestehend aus Blut und Lebergewebe eines Subjekts.

2. Verfahren nach Anspruch 1, wobei die Methylierung mit einem Primer-Set detektiert oder identifiziert wird, das ausgewählt ist aus der Gruppe bestehend aus: einem Primer-Set aus SEQ ID NO: 8 und SEQ ID NO: 9, und/oder einem Primer-Set aus SEQ ID NO: 10 und SEQ ID NO: 11 für humanes SALL3c Gen; einem Primer-Set aus SEQ ID NO: 12 und SEQ ID NO: 13, und/oder einem Primer-Set aus SEQ ID NO: 14 und SEQ ID NO: 15 für humanes ECEL1 Gen; einem Primer-Set aus SEQ ID NO: 16 und SEQ ID NO: 17, und/oder einem Primer-Set aus SEQ ID NO: 18 und SEQ ID NO: 19 für humanes FOXC1 Gen; einem Primer-Set aus SEQ ID NO: 20 und SEQ ID NO: 21, und/oder einem Primer-Set aus SEQ ID NO: 22 und SEQ ID NO: 23 für humanes NRG3 Gen; einem Primer-Set aus SFQ ID NO: 24 und SEQ ID NO: 25, und/oder einem Primer-Set aus SEQ ID NO: 26 und SEQ ID NO: 27 für humanes KCNIP2 Gen; einem Primer-Set aus SEQ ID NO: 28 und SEQ ID NO: 29, und/oder einem Primer-Set aus SEQ ID NO: 30 und SEQ ID NO: 31 für die durch SEQ ID NO: 6 repräsentierte Genregion; einem Primer-Set aus SEQ ID NO: 32 und SEQ ID NO: 33, und/oder einem Primer-Set aus SEQ ID NO: 34 und SEQ ID NO: 35 für die durch SEQ ID NO: 7 repräsentierte Genregion.

3. Verwendung eines Polynukleotids für die in-vitro Diagnose von Leberkrebs, dadurch charakterisiert, dass das Polynukleotid die Methylierung von wenigstens einem der Gene oder Genregionen detektieren kann, das/die ausgewählt ist/sind aus der Gruppe bestehend aus:
(1) humanem SALL3c Gen,
(2) humanem ECEL1 Gen,
(3) humanem FOXC1 Gen,
(4) humanem N RG3 Gen,
(5) humanem KCNIP2 Gen,
(6) SEQ ID NO: 6 (NT_037622.5, 1393861 bis 1395960), und
(7) SEQ ID NO: 7 (NT_022135.15, 7774305 bis 7776805)
das/die detektierbar ist unter Vewendung eines Primer-Sets aus SEQ ID NO: 8 und SEQ ID NO: 9 für humanes SALL3c Gen, eines Primer-Sets aus SEQ ID NO: 12 and SEQ ID NO: 13 für humanes ECEL1 Gen, eines Primer-Sets aus SEQ ID NO: 16 und SEQ ID NO: 17 für humanes FOXC1 Gen, eines Primer-Sets aus SEQ ID NO: 20 und SEQ ID NO: 21 für humanes NRG3 Gen, eines Primer-Sets aus SEQ ID NO: 24 und SEQ ID NO: 25 für humanes KCNIP2 Gen, eines Primer-Sets aus SEQ ID NO: 28 und SEQ ID NO: 29 für SEQ ID NO: 6, und eines Primer-Sets aus SEQ ID NO: 32 und SEQ ID NO: 33 für SEQ ID NO: 7.

4. Verwendung eines Kits für die in-vitro Diagnose von Leberkrebs, dadurch charakterisiert, dass der Kit ein oder mehrere Polynukleotide umfasst, das/die die Methylierung von wenigstens einem der Gene oder Genregionen detektieren kann/können, das/die ausgewählt ist aus der Gruppe bestehend aus:
(1) humanem SALL3c Gen,
(2) humanem ECEL1 Gen,
(3) humanem FOXC1 Gen,
(4) humanem NRG3 Gen,
(5) humanem KCNIP2 Gen,
(6) SEQ ID NO: 6 (NT_037622.5, 1393861 bis 1395960), und
(7) SEQ ID NO: 7 (NT_022135.15, 7774305 bis 7776805),
das/die detektierbar ist unter Verwendung eines Primer-Sets aus SEQ ID NO: 8 und SEQ ID NO: 9 für humanes SALL3c Gen, eines Primer-Sets aus SEQ ID NO: 12 und SEQ ID NO: 13 für humanes ECEL1 Gen, eines Primer-Sets aus SEQ ID NO: 16 und SEQ ID NO: 17 für humanes FOXC1 Gen, eines Primer-Sets aus SEQ ID NO: 20 und SEQ ID NO: 21 für humanes NRG3 Gen, eines Primer-Sets aus SEQ ID NO: 24 und SEQ ID NO: 25 für humanes KCNIP2 Gen, eines Primer-Sets aus SEQ ID NO: 28 und SEQ ID NO: 29 für SEQ ID NO: 6, und eines Primer-Sets aus SEQ ID NO: 32 und SEQ ID NO: 33 für SEQ ID NO: 7.

5. Verwendung eines Mikroarrays für die in-vitro Diagnose von Leberkrebs, dadurch charakterisiert, dass der Mikroarray ein oder mehrere Polynukleotide umfasst, das/die die Methylierung von wenigstens einem der Gene oder Genregionen detektieren kann/können, das/die ausgewählt ist aus der Gruppe bestehend aus:
(1) humanem SALL3c Gen,
(2) humanem ECEL1 Gen,
(3) humanem FOXC1 Gen,
(4) humanem NRG3 Gen,
(5) humanem KCNIP2 Gen,
(6) SEQ ID NO: 6 (NT_037622.5, 1393861 bis 1395960), und
(7) SEQ ID NO: 7 (NT_022135.15, 7774305 bis 7776805),
das/die detektierbar ist unter Verwendung eines Primer-Sets aus SEQ ID NO: 8 und SEQ ID NO: 9 für humanes SALL3c Gen, eines Primer-Sets aus SEQ ID NO: 12 und SEQ ID NO: 13 für humanes ECEL1 Gen, eines Primer-Sets aus SEQ ID NO: 16 und SEQ ID NO: 17 für humanes FOXC1 Gen, eines Primer-Sets aus SEQ ID NO: 20 und SEQ ID NO: 21 für humanes NRG3 Gen, eines Primer-Sets aus SEQ ID NO: 24 und SEQ ID NO: 25 für humanes KCNIP2 Gen, eines Primer-Sets aus SEQ ID NO: 28 und SEQ ID NO: 29 für SEQ ID NO: 6, und eines Primer-Sets aus SEQ ID NO: 32 und SEQ ID NO: 33 für SEQ ID NO: 7.

6. Verwendung gemäß einem der Ansprüche 3 bis 5, wobei das Polynucleotid ausgewählt ist aus der Gruppe bestehend aus: einem Primer-Set aus SEQ ID NO: 8 und SEQ ID NO: 9, und/oder einem Primer-Set aus SEQ ID NO: 10 und SEQ ID NO: 11 für humanes SALL3c Gen; einem Primer-Set aus SEQ ID NO: 12 und SEQ ID NO: 13, und/oder einem Primer-Set aus SEQ ID NO: 14 und SEQ ID NO: 15 für humanes ECEL1 Gen; einem Primer-Set aus SEQ ID NO: 16 und SEQ ID NO: 17, und/oder einem Primer-Set aus SEQ ID NO: 18 und SEQ ID NO: 19 für humanes FOXC1 Gen, einem Primer-Set aus SEQ ID NO: 20 und SEQ ID NO: 21, und/oder einem Primer-Set aus SEQ ID NO: 22 und SEQ ID NO: 23 für humanes NISCG3 Gen; einem Primer-Set aus SEQ ID NO: 24 und SEQ ID NO: 25, und/oder einem Primer-Set aus SEQ ID NO: 26 und SEQ ID NO: 27 für humanes KCNIP22 Gen, einem Primer-Set aus SEQ ID NO: 28 und SEQ ID NO: 29, und/oder einem Printer-Set aus SEQ ID NO: 30 und SEQ ID NO: 31 für die durch SEQ ID NO: 6 repräsentierte Genregion; einem Primer-Set aus SEQ ID NO: 32 und SEQ ID NO: 33, und/oder einem Primer-Set aus SEQ ID NO: 34 und SEQ ID NO: 35 für die durch SEQ ID NO: 7 repräsentierte Generegion.

7. Verwendung gemäß einem der Anspruche 3 bis 6, wobei das Gen oder die Genregion ausgewählt ist aus der Gruppe bestehend aus:
(1) humanem SALL3c Gen,
(2) humanem ECEL1 Gen, und
(3) SEQ ID NO: 6 (NT_037622.5,1393861 bis 1395960).

8. Verwendung eines Polynukleotids gemäß einem der Ansprüche 4 oder 5 zur Herstellung eines Nachweiskits oder Mikroarrays für den Nachweis von Leberkrebs.

9. Primer-Set ausgewählt aus der Gruppe bestehend aus: einem Primer-Set aus SEQ ID NO: 8 und SEQ ID NO: 9 für humanes SALL3c Gen, einem Primer-Set aus SEQ ID NO: 10 und SEQ ID NO: 11 für humanes SALL3c Gen, einem Primer-Set aus SEQ ID NO: 12 und SEQ ID NO: 13 für humanes ECEL1. Gen, einem Primer-Set aus SEQ ID NO:14 und SEQ ID NO: 15 für humanes ECEL1 Gen, einem Primer-Set aus SEQ ID NO: 16 und SEQ ID NO: 17 für humanes FOXC1 Gen, einem Primer-Set aus SEQ ID NO: 18 und SEQ ID NO: 19 für humanes FOXC1 Gen, einem Primer-Set aus SEQ ID NO: 20 und SEQ ID NO: 21 für humanes NRG3 Gen, einem Primer-Set aus SEQ ID NO: 22 und SEQ ID NO: 23 für humanes NRG3 Gen, einem Primer-Set aus SEQ ID NO: 24 und SEQ ID NO: 25 für humanes KCNIP2 Gen, einem Primer-Set aus SEQ ID NO: 26 und SEQ ID NO: 27 für humanes KCNIP2 Gen; einem Primer-Set aus SEQ ID NO: 28 und SEQ ID NO: 29 für die durch SEQ ID NO: 6 repräsentierte Genregion, einem Primer-Set aus SEQ ID NO: 30 und SEQ ID NO: 31 für die durch SEQ ID NO: 6 repräsentierte Genregion, einem Primer-Set aus SEQ ID NO: 32 und SEQ ID NO: 33 für die durch SEQ ID NO: 7 repräsentierte Genregion, und einem Primer-Set aus SEQ ID NO: 34 und SEQ ID NO: 35 für die durch SEQ ID NO: 7 repräsentierte Genregion.

10. Verfahren zur Detektion eines Rückfallrisikos nach einer Leberkrebs-Operation, bei dem in einer Probe das Vorhandensein der Methylierung eines oder mehrerer Gene oder Genregionen nachgewiesen wird, das/die ausgewählt ist/sind aus der Gruppe bestehend aus humanem SALL3c Gen und humanem ECEL1 Gen, und einer durch SEQ ID NO: 6 (NT_037622.5, 1393861 bis 1395960) repräsentierten Genregion, das/die detektierbar ist unter Verwendung eines Primer-Sets aus SEQ ID NO: 8 und SEQ ID NO: 9 für humanes SALL3c Gen, eines Primer-Sets aus SEQ ID NO: 12 und SEQ ID NO: 13 für humanes ECEL1 Gen, und eines Primer-Sets aus SEQ ID NO: 28 und SEQ ID NO: 29, wobei die Probe ausgewählt ist aus der Gruppe bestehend aus Blut und Lebergewebe eines Subjekts.

11. Verfahren gemäß Anspruch 10, wobei die Methylierung mit einem Primer-Set detektiert oder identifiziert wird, das ausgewählt ist aus der Gruppe bestehend aus: einem Primer-Set aus SEQ ID NO: 8 und SEQ ID NO: 9, und/oder einem Primer-Set aus SEQ ID NO: 10 und SEQ ID NO: 11 für humanes SALL3c Gen; einem Primer-Set aus SEQ ID NO: 12 und SEQ ID NO: 13, und/oder einem Primer-Set aus SEQ ID NO: 14 und SEQ ID NO: 15 für humanes ECEL1 Gen; und einem Primer-Set aus SEQ ID NO: 28 und SEQ ID NO: 29, und/oder einem Primer-Set aus SEQ ID NO: 30 und SEQ ID NO: 31 für die durch SEQ ID NO: 6 repräsentierte Genregion.

12. Verwendung eines Polynukleotids für die in-vitro Diagnose eines Rückfallrisikos nach einer Leberkrebs-Operation, dadurch charakterisiert, dass das Polynukleotid die Methylierung von wenigstens einem der Gene oder Genregionen detektieren kann, das/die ausgewählt ist aus der Gruppe bestehend aus humanem SALL3c Gen, humanem ECEL1 Gen, und/oder der Methylierung der Genregionen SEQ ID NO: 6 (NT_037622.5,1393861 bis 1395960), das/die detektierbar ist unter Verwendung eines Primer-Sets aus SEQ ID NO: 8 und SEQ ID NO: 9 für humanes SALL3c Gen, eines Primer-Sets aus SEQ ID NO: 12 und SEQ ID NO: 13 für humanes ECEL1 Gen, und eines Primer-Sets aus SEQ ID NO: 28 und SEQ ID NO: 29 für die durch SEQ ID NO: 6 repräsentierte Genregion.

13. Verwendung eines Kits für die in-vitro Diagnose eines Rückfallrisikos nach einer Leberkrebs-Operation, dadurch charakterisiert, dass der Kit ein oder mehrere Polynukleotide umfasst, das/die die Methylierung von wenigstens einem der Gene oder Genregionen nachweisen kann/können, das/die ausgewählt ist aus der Gruppe bestehend aus humanem SALL3c Gen, humanem ECEL1 Gen, und/oder der Methylierung der Genregionen SEQ ID NO: 6 (NT_037622.5, 1393861 bis 1395960), das/die detektierbar ist unter Verwendung eines Primer-Sets aus SEQ ID NO: 8 und SEQ ID NO: 9 für humanes SALL3c Gen, eines Primer-Sets aus SEQ ID NO: 12 und SEQ ID NO: 13 für humanes ECEL1 Gen, und eines Primer-Sets aus SEQ ID NO: 28 und SEQ ID NO: 29 für die durch SEQ ID NO: 6 repräsentierte Genregion.

14. Verwendung eines Mikroarrays für die in-vitro Diagnose eines Rückfallrisikos nach einer Leberkrebs-Operation, dadurch charakterisiert, dass der Mikroarray ein oder mehrere Polynukleotide umfasst, das/die die Methylierung von wenigstens einem der Gene oder Genregionen nachweisen kann/können, das/die ausgewählt ist aus der Gruppe bestehend aus humanem SALL3c Gen, humanem ECEL1 Gen, und/oder der Methylierung der Genregionen SEQ ID NO: 6 (NT_037622.5, 1393861 bis 1395960), das/die detektierbar ist unter Verwendung eines Primer-Sets aus SEQ ID NO:8 und SEQ ID NO:9 für humanes SALL3c Gen, eines Primer-Sets aus SEQ ID NO: 12 und SEQ ID NO: 13 für humanes ECEL1 Gen, und eines Primer-Sets aus SEQ ID NO: 28 und SEQ ID NO: 29 für die durch SEQ ID NO: 6 repräsentierten Genregion.

15. Verwendung gemäß einem der Ansprüche 12 bis 14, wobei das Polynukleotid ausgewählt ist aus der Gruppe bestehend aus: einem Primer-Set aus SEQ ID NO: 8 und SEQ ID NO: 9, und/oder einem Primer-Set aus SEQ ID NO: 10 und SEQ ID NO: 11 für humanes SALL3c Gen; einem Primer-Set aus SEQ ID NO: 12 und SEQ ID NO: 13, und/oder einem Primer-Set aus SEQ ID NO: 14 und SEQ ID NO: 15 für humanes ECEL1 Gen; und einem Primer-Set aus SEQ ID NO:28 und SEQ ID NO: 29, und/oder einem Primer-Set aus SEQ ID NO: 30 und SEQ ID NO: 31 für die durch SEQ ID NO: 6 repräsentierte Genregion.

16. Verwendung eines Polynukleotids nach einem der Ansprüche 13 oder 14 zur Herstellung eines Detektionskits oder Mikroarrays für den Nachweis des Rückfallrisikos nach einer Leberkrebs-Operation.

## Revendications

1. Procédé pour la détection d'un cancer du foie en détectant dans un échantillon la présence d'une méthylation dans l'un quelconque ou plusieurs gènes ou régions génétiques choisis dans le groupe constitué par le gène humain SALL3c, le gène humain ECEL1, le gène humain FOXC1, le gène humain NRG3 et le gène humain KCNIP2, une région génétique représentée par la SEQ ID NO: 6 (NT_037622.5, 1393861 à 1395960) et une région génétique représentée par la SEQ ID NO: 7 (NT_022135.15, 7774305 à 7776805) qui peut être détectée en utilisant une série d'amorces de la SEQ ID NO: 8 et la SEQ ID NO: 9 pour le gène humain SALL3c, une série d'amorces de la SEQ ID NO: 12 et la SEQ ID NO: 13 pour le gène humain ECEL1, une série d'amorces de la SEQ ID NO: 16 et la SEQ ID NO: 17 pour le gène humain FOXC1, une série d'amorces de la SEQ ID NO: 20 et la SEQ ID NO: 21 pour le gène humain NRG3, une série d'amorces de la SEQ ID NO: 24 et la SEQ ID NO: 25 pour le gène humain KCNIP2, une série d'amorces de la SEQ ID NO: 28 et la SEQ ID NO: 29 pour la région génétique représentée par la SEQ ID NO: 6, et une série d'amorces de la SEQ ID NO: 32 et la SEQ ID NO: 33 pour la région génétique représentée par la SEQ ID NO: 7, l'échantillon étant sélectionné dans le groupe constitué de sang et de tissue de foie du sujet.

2. Procédé selon la revendication 1, dans lequel la méthylation est détectée ou identifiée par une série d'amorces choisies dans le groupe constitué par : une série d'amorces de la SEQ ID NO: 8 et la SEQ ID NO: 9, et/ou une série d'amorces de la SEQ ID NO: 10 et la SEQ ID NO: 11 pour le gène humain SALL3c : une série d'amorces de la SEQ ID NO: 12 et la SEQ ID NO: 13, et/ou une série d'amorces de la SEQ ID NO: 14 et la SEQ ID NO: 15 pour le gène humain ECEL1 ; une série d'amorces de la SEQ ID NO: 16 et la SEQ ID NO: 17, et/ou une série d'amorces de la SEQ ID NO: 18 et la SEQ ID NO: 19 pour le gène humain FOXC1 ; une série d'amorces de la SEQ ID NO: 20 et de la SEQ ID NO: 21, et/ou une série d'amorces de la SEQ ID NO: 22 et la SEQ ID NO: 23 pour le gène humain NRG3 ; une série d'amorces de la SEQ ID NO: 24 et la SEQ ID NO: 25, et/ou une série d'amorces de la SEQ ID NO: 26 et la SEQ ID NO: 27 pour le gène humain KCNIP2 ; une série d'amorces de la SEQ ID NO: 28 et la SEQ ID NO: 29, et/ou une série d'amorces de la SEQ ID NO: 30 et la SEQ ID NO: 31 pour la région génétique représentée par la SEQ ID NO: 6 ; une série d'amorces de la SEQ ID NO: 32 et la SEQ ID NO: 33, et/ou une série d'amorces de la SEQ ID NO: 34 et la SEQ ID NO: 35 pour la région génétique représentée par la SEQ ID NO: 7.

3. Utilisation d'un polynucléotide pour le diagnostic in-vitro d'un cancer du foie, **caractérisée en ce que** le polynucléotide peut détecter la méthylation d'au moins un des gènes ou régions génétiques choisis dans le groupe composé de :
(1) gène humain SALL3c,
(2) gène humain ECEL1,
(3) gène humain FOXC1,
(4) gène humain NRG3,
(5) gène humain KCNIP2,
(6) SEQ ID NO: 6 (NT_037622.5, 1393861 to 1395960), et
(7) SEQ ID NO: 7 (NT_022135.15, 7774305 à 7776805)
laquelle peut être détectée en utilisant une série d'amorces de la SEQ ID NO: 8 et la SEQ ID NO: 9 pour le gène humain SALL3c, une série d'amorces de la SEQ ID NO: 12 et la SEQ ID NO: 13 pour le gène humain ECEL1, une série d'amorces de la SEQ ID NO: 16 et la SEQ ID NO: 17 pour le gène humain FOXC1, une série d'amorces de la SEQ ID NO: 20 et la SEQ ID NO: 21 pour le gène humain NRG3, une série d'amorces de la SEQ ID NO: 24 et la SEQ ID NO: 25 pour le gène humain KCNIP2, une série d'amorces de la SEQ ID NO: 28 et la SEQ ID NO: 29 pour la SEQ ID NO: 6, et une série d'amorces de la SEQ ID NO: 32 et la SEQ ID NO: 33 pour la SEQ ID NO: 7.

4. Utilisation d'une trousse de diagnostic in-vitro d'un cancer du foie, **caractérisée en ce que** la trousse comprend un ou plusieurs polynucléotides qui peuvent détecter la méthylation d'au moins un des gènes ou régions génétiques choisis dans le groupe composé de :
(1) gène humain SALL3c,
(2) gène humain ECEL1,
(3) gène humain FOXC1,
(4) gène humain NRG3,
(5) gène humain KCNIP2,
(6) SEQ ID NO: 6 (NT_037622.5, 1393861 à 1395960), et
(7) SEQ ID NO: 7 (NT_022135.15, 7774305 à 7776805)
laquelle peut être détectée en utilisation une série d'amorces de la SEQ ID NO: 8 et la SEQ ID NO: 9 pour le gène humain SALL3c, une série d'amorces de la SEQ ID NO: 12 et la SEQ ID NO: 13 pour le gène humain ECEL1, une série d'amorces de la SEQ ID NO: 16 et la SEQ ID NO: 17 pour le gène humain FOXC1, une série d'amorces de la SEQ ID NO: 20 et la SEQ ID NO: 21 pour le gène humain NRG3, une série d'amorces de la SEQ ID NO: 24 et la SEQ ID NO: 25 pour le gène humain KCNIP2, une série d'amorces de la SEQ ID NO: 28 et la SEQ ID NO: 29 pour la SEQ ID NO: 6, et une série d'amorces de la SEQ ID NO: 32 et la SEQ ID NO: 33 pour la SEQ ID NO: 7.

5. Utilisation d'une puce à ADN pour un diagnostic in-vitro d'un cancer du foie, **caractérisée en ce que** la puce à ADN comprend un ou plusieurs polynucléotides qui peuvent détecter la méthylation d'au moins un des gènes ou régions génétiques choisis dans le groupe constitué de :
(1) gène humain SALL3c,
(2) gène humain ECEL1,
(3) gène humain FOXC1,
(4) gène humain NRG3,
(5) gène humain KCNIP2,
(6) SEQ ID NO: 6 (NT_037622.5, 1393861 à 1395960), et
(7) SEQ ID NO: 7 (NT_022135.15, 7774305 à 7776805)
laquelle peut être détectée en utilisant une série d'amorces de la SEQ ID NO: 8 et la SEQ ID NO: 9 pour le gène humain SALL3c, une série d'amorces de la SEQ ID NO: 12 et la SEQ ID NO: 13 pour le gène humain ECEL1, une série d'amorces de la SEQ ID NO: 16 et la SEQ ID NO: 17 pour le gène humain FOXC1, une série d'amorces de la SEQ ID NO: 20 et la SEQ ID NO: 21 pour le gène humain NRG3, une série d'amorces de la SEQ ID NO: 24 et la SEQ ID NO: 25 pour le gène humain KCNIP2, une série d'amorces de la SEQ ID NO: 28 et la SEQ ID NO: 29 pour la SEQ ID NO: 6, et une série d'amorces pour la SEQ ID NO: 32 et la SEQ ID NO: 33 pour la SEQ ID NO: 7.

6. Utilisation selon l'une des revendications 3 à 5, dans laquelle le polynucléotide est choisi dans le groupe composé de : une série d'amorces de la SEQ ID NO: 8 et la SEQ ID NO: 9, et/ou une série d'amorces de la SEQ ID NO: 10 et la SEQ ID NO:11 pour le gène humain SALL3c ; une série d'amorces de la SEQ ID NO: 12 et la SEQ ID NO: 13, et/ou une série d'amorces de la SEQ ID NO: 14 et la SEQ ID NO: 15 pour le gène humain ECEL1 ; une série d'amorces de la SEQ ID NO: 16 et la SEQ ID NO: 17, et/ou une série d'amorces de la SEQ ID NO: 18 et la SEQ ID NO: 19 pour le gène humain FOXC1 ; une série d'amorces de la SEQ ID NO: 20 et la SEQ ID NO: 21, et/ou une série d'amorces de la SEQ ID NO: 22 et la SEQ ID NO: 23 pour le gène humain NRG3 ; une série d'amorces de la SEQ ID NO: 24 et la SEQ ID NO: 25, et/ou une série d'amorces de la SEQ ID NO: 26 et la SEQ ID NO: 27 pour le gène humain KCNIP2, une série d'amorces de la SEQ ID NO: 28 et la SEQ ID NO: 29, et/ou une série d'amorces de la SEQ ID NO: 30 et la SEQ ID NO: 31 pour la région génétique représentée par la SEQ ID NO: 6 ; une série d'amorces de la SEQ ID NO: 32 et la SEQ ID NO: 33, et/ou une série d'amorces de la SEQ ID NO: 34 et la SEQ ID NO: 35 pour la région génétique représentée par la SEQ ID NO: 7.

7. Utilisation selon l'une des revendications 3 à 6, dans laquelle le gène ou la région génétique est choisi dans le groupe composé de :
(1) gène humain SALL3c,
(2) gène humain ECEL1, et
(3) SEQ ID NO: 6 (NT_037622.5, 1393861 à 1395960).

8. Utilisation d'un polynucléotide selon la revendication 4 ou 5 pour la fabrication d'une trousse de détection ou d'une puce à ADN pour détection un cancer du foie.

9. Une série d'amorces choisies dans le groupe composé de : une série d'amorces de la SEQ ID NO: 8 et la SEQ ID NO: 9 pour le gène humain SALL3c, une série d'amorces de la SEQ ID NO: 10 et la SEQ ID NO: 11 pour le gène humain SALL3c, une série d'amorces de la SEQ ID NO: 12 et la SEQ ID NO: 13 pour le gène humain ECEL1, une série d'amorces de la SEQ ID NO: 14 et la SEQ ID NO: 15 pour le gène humain ECEL1, une série d'amorces de la SEQ ID NO: 16 et la SEQ ID NO: 17 pour le gène humain FOXC1, une série d'amorces de la SEQ ID NO: 18 et la SEQ ID NO: 19 pour le gène humain FOXC1, une série d'amorces de la SEQ ID NO: 20 et la SEQ ID NO: 21 pour le gène humain NRG3, une série d'amorces de la SEQ ID NO: 22 et la SEQ ID NO: 23 pour le gène humain NRG3, une série d'amorces de la SEQ ID NO: 24 et la SEQ ID NO: 25 pour le gène humain KCNIP2, une série d'amorces de la SEQ ID NO: 26 et la SEQ ID NO: 27 pour le gène humain KCNIP2 ; une série d'amorces de la SEQ ID NO: 28 et la SEQ ID NO: 29 pour la région génétique représentée par la SEQ ID NO: 6, une série d'amorces de la SEQ ID NO: 30 et la SEQ ID NO: 31 pour la région génétique représentée par la SEQ ID NO: 6, une série d'amorces de la SEQ ID NO: 32 et la SEQ ID NO: 33 pour la région génétique représentée par la SEQ ID NO: 7, et une série d'amorces de la SEQ ID NO: 34 et la SEQ ID NO: 35 pour la région génétique représentée par la SEQ ID NO: 7.

10. Procédé de détection d'un risque de récidive après une opération d'un cancer du foie en détectant dans un échantillon la présence de méthylation de l'un quelconque ou de plusieurs gènes ou régions génétiques choisis dans le groupe constitué du gène humain SALL3c et du gène humain ECEL1, et une région génétique représentée par la SEQ ID NO: 6 (NT_037622.5, 1393861 à 1395960), laquelle peut être détectée en utilisant une série d'amorces de la SEQ ID NO: 8 et la SEQ ID NO: 9 pour le gène humain SALL3c, une série d'amorces de la SEQ ID NO: 12 et la SEQ ID NO: 13 pour le gène humain ECEL1, et une série d'amorces de la SEQ ID NO: 28 et la SEQ ID NO: 29, l'échantillon étant choisi dans le groupe composé de sang et de tissu du foie du sujet.

11. Procédé selon la revendication 10, dans lequel la méthylation est détectée ou identifiée par une série d'amorces choisies dans le groupe composé de : une série d'amorces de la SEQ ID NO: 8 et la SEQ ID NO: 9, et/ou une série d'amorces de la SEQ ID NO: 10 et la SEQ ID NO: 11 pour le gène humain SALL3c ; une série d'amorces de la SEQ ID NO: 12 et la SEQ ID NO: 13, et/ou une série d'amorces de la SEQ ID NO: 14 et la SEQ ID NO: 15 pour le gène humain ECEL1 ; et une série d'amorces de la SEQ ID NO: 28 et la SEQ ID NO: 29, et/ou une série d'amorces de la SEQ ID NO: 30 et la SEQ ID NO: 31 pour la région génétique représentée par la SEQ ID NO: 6.

12. Utilisation d'un polynucléotide pour le diagnostic in-vitro d'un risque de récidive après une opération d'un cancer du foie, **caractérisée en ce que** le polynucléotide peut détecter la méthylation d'au moins un des gènes ou régions génétiques choisis dans le groupe composé du gène humain SALL3c, du gène humain ECEL1, et/ou la méthylation des régions génétiques SEQ ID NO: 6 (NT_037622.5, 1393861 à 1395960), laquelle peut être détectée en utilisant une série d'amorces de la SEQ ID NO: 8 et la SEQ ID NO: 9 pour le gène humain SALL3c, une série d'amorces de la SEQ ID NO: 12 et la SEQ ID NO: 13 pour le gène humain ECEL1, et une série d'amorces de la SEQ ID NO: 28 et la SEQ ID NO: 29 pour la région génétique représentée par la SEQ ID NO: 6.

13. Utilisation d'une trousse pour le diagnostic in-vitro d'un risque de récidive après une opération d'un cancer du foie, **caractérisée en ce que** la trousse comprend un ou plusieurs nucléotides qui peuvent être détecter la méthylation d'au moins un des gènes ou régions génétiques choisis dans le groupe composé du gène humain SALL3c, du gène humain ECEL1, et/ou la méthylation des régions génétiques SEQ ID NO: 6 (NT_037622.5, 1393861 à 1395960), laquelle peut être détectée en utilisant une série d'amorces de la SEQ ID NO: 8 et la SEQ ID NO: 9 pour le gène humain SALL3c, une série d'amorces de la SEQ ID NO: 12 et la SEQ ID NO: 13 pour le gène humain ECEL1, et une série d'amorces de la SEQ ID NO: 28 et la SEQ ID NO: 29 pour la région génétique représentée par la SEQ ID NO: 6.

14. Utilisation d'une puce à ADN pour le diagnostic in-vitro d'un risque de récidive après une opération d'un cancer du foie, **caractérisée en ce que** la puce à ADN comprend un ou plusieurs polynucléotides qui peuvent détecter la méthylation d'au moins un des gènes ou régions génétiques choisis dans le groupe composé du gène humain SALL3c, du gène humain ECEL1, et/ou la méthylation des régions génétiques SEQ ID NO: 6 (NT_037622.5, 1393861 à 1395960), laquelle peut être détectée en utilisant une série d'amorces de la SEQ ID NO: 8 et la SEQ ID NO: 9 pour le gène humain SALL3c, une série d'amorces de la SEQ ID NO: 12 et la SEQ ID NO: 13 pour le gène humain ECEL1, et une série d'amorces de la SEQ ID NO: 28 et la SEQ ID NO: 29 pour la région génétique représentée par la SEQ ID NO: 6.

15. Utilisation selon l'une des revendications 12 à 14, dans laquelle le polynucléotide est choisi dans le groupe composé de : une série d'amorces de la SEQ ID NO: 8 et la SEQ ID NO: 9, et/ou une série d'amorces de la SEQ ID NO: 10 et la SEQ ID NO: 11 pour le gène humain SALL3c ; une série d'amorces de la SEQ ID NO: 12 et la SEQ ID NO: 13, et/ou une série d'amorces de la SEQ ID NO: 14 et la SEQ ID NO: 15 pour le gène humain ECEL1 ; et une série d'amorces de la SEQ ID NO: 28 et la SEQ ID NO: 29, et/ou une série d'amorces de la SEQ ID NO: 30 et la SEQ ID NO: 31 pour la région génétique représentée par la SEQ ID NO: 6.

16. Utilisation d'un polynucléotide selon la revendication 13 ou 14 pour la fabrication d'une trousse de détection ou d'une puce à ADN pour la détection d'un risque de récidive après une opération d'un cancer du foie.


### SEQUENCE LISTING

<110> The university of Tokyo Japanese Foundation For Cancer Research
<120> Gene associated with liver cancer, and method for determination of the risk of acquiring liver cancer
<130> 128078001
<140> PCT/JP20008/003082
   <141> 2008-10-29
<150> JP20070281552
   <151> 2007-10-30
<160> 35
<170> PatentIn version 3.3
<210> 1
   <211> 3713
   <212> DNA
   <213> Homo sapiens
<400> 1
   agcgccgcta gcagcatgtc tcggcgcaag caggccaagc cccagcacct caagtcggac      60
   gaggagctgc tgccgcctga cggggctccc gagcacgccg ccccggggga aggtgcggag     120
   gacgcagaca gcgggcccga gagccgcagc gggggcgagg agaccagcgt gtgcgagaaa     180
   tgctgcgccg agttcttcaa gtgggcggac ttcctggagc accagcggag ctgcaccaag     240
   ctcccgcccg tgctgatcgt gcacgaggac gcgcccgcgc cgccccccga ggacttcccc      300
   gagccttcgc ccgccagctc ccccagcgag cgcgccgaaa gcgaggcggc cgaggaggcg     360
   ggtgcggagg gcgcggaggg tgaggccagg ccggtggaga aggaggccga gcccatggac     420
   gcggaacccg cgggggacac gcgcgcgccc cggcccccgc ctgcggcccc tgcaccccca     480
   acgcccgcct acggcgcgcc cagcaccaac gtgaccctgg aggcgctgct gagcaccaag     540
   gtggcggtgg cgcagttctc gcagggcgcg cgcgcggcag gcggctcggg agcaggtgga     600
   ggcgtggcag ctgcagccgt gcccctgatc ctggaacagc tcatggccct gcagcagcag     660
   cagatccacc agctgcagct catcgagcag atccgcagcc aggtggccct catgcagcgc     720
   ccgccgccgc ggccctcact cagccccgcg gccgccccga gcgcaccggg cccggccccc     780
   agccagctgc ccgggctggc cgcgctcccg ctgtcggccg gggcccctgc cgccgccatc      840
   gcgggctcgg gccccgccgc cccggccgcc ttcgagggcg cgcagccgct gtcccggccc     900
   gagtctggcg ccagcacccc cggcggccct gcggagccca gcgcgcccgc cgcccccagc      960
   gccgcccctg cccccgctgc ccccgccccg gcgccagcgc cgcagagcgc ggcctcgtcg    1020
   cagccgcaga gcgcatccac gccgcctgcc ctggccccgg ggtccctgct gggtgcggcg    1080
   cccggcctgc caagtccgct tctacctcag acttccgcca gcggcgtcat cttccccaac    1140
   ccgctggtca gcatcgcggc cacggccaac gctctggacc cgctgtccgc gctcatgaag    1200
   caccgcaagg gcaagccgcc caatgtgtcg gtgttcgagc ccaaagccag cgccgaggac    1260
   ccgttcttca agcacaaatg ccgcttctgc gccaaggtct tcggcagcga cagcgcgctc    1320
   cagatccacc tgcgctcgca cacaggcgag cggcccttca agtgcaacat ctgcgggaac    1380
   cgcttctcca ccaaaggcaa cctgaaggtg cacttccaga ggcacaagga gaagtacccc    1440
   cacatccaga tgaaccctta cccggtcccc gagtacctgg acaacgtgcc cacctgctcg    1500
   ggcatcccct acggcatgtc gctgcccccc gagaagcccg tgaccacctg gctggacagc 1560
   aagcccgtgc tgcccaccgt gcccacgtcc gtggggctgc aactgccgcc cgctgtccct 1620
   ggcgcgcacg gctacgccga ctctcccagc gccaccccag ccagccgctc cccgcagagg 1680
   ccctcgcccg cctccagcga gtgcgcctcc ttgtccccag gcctcaacca cgtggagtcc 1740
   ggcgtgtcgg ccaccgccga gtccccacag tcgctcctcg gcgggccgcc cgtcactaaa 1800
   gccgagcccg tcagcctgcc ctgcaccaac gccagggccg gggacgctcc cgtgggcgcg 1860
   caggctagcg ctgcacccac atcggtggac ggcgcaccca cgagcctcgg cagccccggg 1920
   ctgcccgccg tctccgagca gttcaaggcc cagtttccgt tcggggggct gctagactcg 1980
   atgcaaacgt cggaaacctc gaagctgcag cagctggtgg agaacatcga caagaagatg 2040
   acggacccga accagtgcgt catctgccac cgggtgctga gctgccagag cgcgctgaag 2100
   atgcactacc ggacgcacac gggggagcgg ccgttcaagt gcaagatctg cggccgcgcc 2160
   ttcaccacca agggcaacct caagacgcac ttcggcgtgc accgtgcaaa gccgcccctg 2220
   cgcgtgcagc actcctgccc catctgccag aagaagttca ccaacgccgt ggtcctgcag 2280
   cagcacatcc gcatgcacat gggcggccag atccccaaca cgccgctgcc ggagggcttc 2340
   caggatgcca tggactccga gctggcctac gacgacaaga acgcggagac cctgagcagc 2400
   tacgatgacg acatggacga gaactccatg gaggacgacg ctgagctgaa ggacgcggcc 2460
   accgacccgg ccaagccact cctgtcctat gcggggtcct gcccgccctc cccgccctcg 2520
   gtcatctcca gcattgccgc cctggagaac cagatgaaga tgatcgactc ggtcatgagc 2580
   tgccagcagc tgaccggcct caagtccgtg gagaacgggt ccggggagag tgaccgcctg 2640
   agcaacgact cctcgtcggc cgtgggcgac ctggagagcc gcagcgcggg cagccccgcc 2700
   ctgtccgagt cctcgtcctc gcaggccctg tcgccggccc ccagcaatgg tgagagcttc 2760
   cgctccaagt ccccgggcct gggcgccccg gaggagcccc aggaaatccc gctcaagacc 2820
   gagaggccgg acagcccagc cgccgccccg ggcagcggag gcgcccctgg ccgcgcgggc 2880
   atcaaggagg aggcgccctt cagcctgctg ttcctgagca gggagcgggg tcccagccaa 2940
   agcactccta gcctgatctc cagcgccgca cccaccatga tcaaaatgga agtgaacggt 3000
   cacggcaagg ccatggcgct gggcgagggt cccccgctgc ccgcgggcgt ccaggtcccc 3060
   gccgggcctc agacagtgat gggcccgggc ctggcgccca tgctggcccc cccaccgcgc 3120
   cggacgccca agcagcacaa ctgccagtcg tgcgggaaga ccttctcctc ggccagcgcc 3180
   ctgcagatcc atgagcgcac gcacaccggc gagaagccgt tcggctgcac catctgcggc 3240
   cgggccttca ccactaaggg caacctcaag gtgcacatgg ggacacacat gtggaataac 3300
   gcccccgcga gacgcggccg ccgcctgtct gtggagaacc ccatggctct cctagggggt 3360
   gatgccctga agttctctga aatgttccag aaggacctgg cagctcgggc aatgaacgtc 3420
   gaccccagtt tttggaacca gtatgctgca gccatcacta acgggctcgc catgaagaac 3480
   aacgagatct ccgtcatcca gaacggcggc atcccccagc tccccgtgag tcttgggggc 3540
   agcgccctcc cccctctggg cagcatggcc agtgggatgg acaaagcacg cactggcagt 3600
   agcccaccca tcgtcagctt ggacaaagcg agctcagaaa cagcagccag ccgcccattc 3660
   acgcggttta tcgaggataa caaggagatt ggtatcaact agccagtgac tcg 3713
<210> 2
   <211> 2872
   <212> DNA
   <213> Homo sapiens
<400> 2
   gggcgctggg agacaccgga cgcccgctcg gctgcgctgc ggctcaggcc cccgctcggg 60
   cccgacccgc tcggtcaccg ccggctcggg cgcgcacctg ccggctgcgg ccccagggcc 120
   atgcggaggc ccacgaggag gccggcggcc acgcgcatcc cgtagcccag gtggcccagg 180
   tctgcaccgc ggcggcctcg gcgccatgga gcccccgtat tcgctgacgg cgcactacga 240
   tgagttccaa gaggtcaagt acgtgagccg ctgcggcgcg gggggcgcgc gcggggcctc 300
   cctgcccccg ggcttcccgt tgggcgctgc gcgcagcgcc accggggccc ggtccgggct 360
   gccgcgctgg aaccggcgcg aggtgtgcct gctgtcgggg ctggtgttcg ccgccggcct 420
   ctgcgccatt ctggcggcta tgctggccct caagtacctg ggcccggtcg cggccggcgg 480
   cggcgcctgt cccgagggct gccctgagcg caaggccttc gcgcgcgccg ctcgcttcct 540
   ggccgccaac ctggacgcca gcatcgaccc atgccaggac ttctactcgt tcgcctgcgg 600
   cggttggctg cggcgccacg ccatccccga cgacaagctc acctatggca ccatcgcggc 660
   catcggcgag caaaacgagg agcgcctacg gcgcctgctg gcgcggcccg ggggtgggcc 720
   tggcggcgcg gcccagcgca aggtgcgcgc cttcttccgc tcgtgcctcg acatgcgcga 780
   gatcgagcga ctgggcccgc gacccatgct agaggtcatc gaggactgcg ggggctggga 840
   cctgggcggc gcggaggagc gtccgggggt cgcggcgcga tgggacctca accggctgct 900
   gtacaaggcg cagggcgtgt acagcgccgc cgcgctcttc tcgctcacgg tcagcctgga 960
   cgacaggaac tcctcgcgct acgtcatccg cattgaccag gatgggctca ccctgccaga 1020
   gaggaccctg tacctcgctc aggatgagga cagtgagaag atcctggcag catacagggt 1080
   gttcatggag cgagtgctca gcctcctggg tgcagacgct gtggaacaga aggcccaaga 1140
   gatcctgcaa gtggagcagc agctggccaa catcactgtg tcagagcatg acgacctacg 1200
   gcgagatgtc agctccatgt acaacaaggt gacgctgggg cagctgcaga agatcacccc 1260
   ccacttgcgg tggaagtggc tgctagacca gatcttccag gaggacttct cagaggaaga 1320
   ggaggtggtg ctgctggcga cagactacat gcagcaggtg tcgcagctca tccgctccac 1380
   accccaccgg gtcctgcaca actacctggt gtggcgcgtg gtggtggtcc tgagtgaaca 1440
   cctgtccccg ccattccgtg aggcactgca cgagctggca caggagatgg agggcagcga 1500
   caagccacag gagctggccc gggtctgctt gggccaggcc aatcgccact ttggcatggc 1560
   gcttggcgcc ctctttgtac atgagcactt ctcagctgcc agcaaagcca aggtgcagca 1620
   gctagtggaa gacatcaagt acatcctggg ccagcgcctg gaggagctgg actggatgga 1680
   cgccgagacc agggctgctg ctcgggccaa gctccagtac atgatggtga tggtcggcta 1740
   cccggacttc ctgctgaaac ccgatgctgt ggacaaggag tatgagtttg aggtccatga 1800
   gaagacctac ttcaagaaca tcttgaacag catccgcttc agcatccagc tctcagttaa 1860
   gaagattcgg caggaggtgg acaagtccac gtggctgctc cccccacagg cgctcaatgc 1920
   ctactatcta cccaacaaga accagatggt gttccccgcg ggcatcctgc agcccaccct 1980
   gtacgaccct gacttcccac agtctctcaa ctacgggggc atcggcacca tcattggaca 2040
   tgagctgacc cacggctacg acgactgggg gggccagtat gaccgctcag ggaacctgct 2100
   gcactggtgg acggaggcct cctacagccg cttcctgcga aaggctgagt gcatcgtccg 2160
   tctctatgac aacttcactg tctacaacca gcgggtgaac gggaaacaca cgcttgggga 2220
   gaacatcgca gatatgggcg gcctcaagct ggcctaccac gcctatcaga agtgggtgcg 2280
   ggagcacggc ccagagcacc cacttccccg gctcaagtac acacatgacc agctcttctt 2340
   cattgccttt gcccagaact ggtgcatcaa gcggcggtcg cagtccatct acctgcaggt 2400
   gctgactgac aagcatgccc ctgagcacta cagggtgctg ggcagtgtgt cccagtttga 2460
   ggagtttggc cgggctttcc actgtcccaa ggactcaccc atgaaccctg cccacaagtg 2520
   ttccgtgtgg tgagcctggc tgcccgcctg cacgccccca ctgcccccgc acgaatcacc 2580
   tcctgctggc taccggggca ggcatgcacc cggtgccagc cccgctctgg gcaccacctg 2640
   ccttccagcc cctccaggac ccggtccccc tgctgcccct cacttcagga ggggcctgga 2700
   gcagggtgag gctggacttt ggggggctgt gagggaaata tactggggtc cccagattct 2760
   gctctaaggg ggccagaccc tctgccaggc tggattgtac gggccccacc ttcgctgtgt 2820
   tcttgctgca aagtctggtc aataaatcac tgcactgtta aaaaaaaaaa aa 2872
<210> 3
   <211> 3452
   <212> DNA
   <213> Homo sapiens
<400> 3
   atgcaggcgc gctactccgt gtccagcccc aactccctgg gagtggtgcc ctacctcggc      60
   ggcgagcaga gctactaccg cgcggcggcc gcggcggccg ggggcggcta caccgccatg     120
   ccggccccca tgagcgtgta ctcgcaccct gcgcacgccg agcagtaccc gggcggcatg     180
   gcccgcgcct acgggcccta cacgccgcag ccgcagccca aggacatggt gaagccgccc     240
   tatagctaca tcgcgctcat caccatggcc atccagaacg ccccggacaa gaagatcacc     300
   ctgaacggca tctaccagtt catcatggac cgcttcccct tctaccggga caacaagcag     360
   ggctggcaga acagcatccg ccacaacctc tcgctcaacg agtgcttcgt caaggtgccg     420
   cgcgacgaca agaagccggg caagggcagc tactggacgc tggacccgga ctcctacaac     480
   atgttcgaga acggcagctt cctgcggcgg cggcggcgct tcaagaagaa ggacgcggtg     540
   aaggacaagg aggagaagga caggctgcac ctcaaggagc cgcccccgcc cggccgccag     600
   cccccgcccg cgccgccgga gcaggccgac ggcaacgcgc ccggtccgca gccgccgccc     660
   gtgcgcatcc aggacatcaa gaccgagaac ggtacgtgcc cctcgccgcc ccagcccctg     720
   tccccggccg ccgccctggg cagcggcagc gccgccgcgg tgcccaagat cgagagcccc     780
   gacagcagca gcagcagcct gtccagcggg agcagccccc cgggcagcct gccgtcggcg     840
   cggccgctca gcctggacgg tgcggattcc gcgccgccgc cgcccgcgcc ctccgccccg     900
   ccgccgcacc atagccaggg cttcagcgtg gacaacatca tgacgtcgct gcgggggtcg     960
   ccgcagagcg cggccgcgga gctcagctcc ggccttctgg cctcggcggc cgcgtcctcg    1020
   cgcgcgggga tcgcaccccc gctggcgctc ggcgcctact cgcccggcca gagctccctc    1080
   tacagctccc cctgcagcca gacctccagc gcgggcagct cgggcggcgg cggcggcggc    1140
   gcgggggccg cggggggcgc gggcggcgcc gggacctacc actgcaacct gcaagccatg    1200
   agcctgtacg cggccggcga gcgcgggggc cacttgcagg gcgcgcccgg gggcgcgggc    1260
   ggctcggccg tggacgaccc cctgcccgac tactctctgc ctccggtcac cagcagcagc    1320
   tcgtcgtccc tgagtcacgg cggcggcggc ggcggcggcg ggggaggcca ggaggccggc   1380
   caccaccctg cggcccacca aggccgcctc acctcgtggt acctgaacca ggcgggcgga   1440
   gacctgggcc acttggcgag cgcggcggcg gcggcggcgg ccgcaggcta cccgggccag   1500
   cagcagaact tccactcggt gcgggagatg ttcgagtcac agaggatcgg cttgaacaac   1560
   tctccagtga acgggaatag tagctgtcaa atggccttcc cttccagcca gtctctgtac   1620
   cgcacgtccg gagctttcgt ctacgactgt agcaagtttt gacacaccct caaagccgaa   1680
   ctaaatcgaa ccccaaagca ggaaaagcta aaggaaccca tcaaggcaaa atcgaaacta   1740
   aaaaaaaaaa atccaattaa aaaaaacccc tgagaatatt caccacacca gcgaacagaa   1800
   tatccctcca aaaattcagc tcaccagcac cagcacgaag aaaactctat tttcttaacc   1860
   gattaattca gagccacctc cactttgcct tgtctaaata aacaaacccg taaactgttt   1920
   tatacagaga cagcaaaatc ttggtttatt aaaggacagt gttactccag ataacacgta   1980
   agtttcttct tgcttttcag agacctgctt tcccctcctc ccgtctcccc tctcttgcct   2040
   tcttccttgc ctctcacctg taagatatta ttttatccta tgttgaaggg agggggaaag   2100
   tccccgttta tgaaagtcgc tttcttttta ttcatggact tgttttaaaa tgtaaattgc   2160
   aacatagtaa tttattttta atttgtagtt ggatgtcgtg gaccaaacgc cagaaagtgt   2220
   tcccaaaacc tgacgttaaa ttgcctgaaa ctttaaattg tgcttttttt ctcattataa   2280
   aaagggaaac tgtattaatc ttattctatc ctcttttctt tctttttgtt gaacatattc   2340
   attgtttgtt tattaataaa ttaccattca gtttgaatga gacctatatg tctggatact   2400
   ttaatagagc tttaattatt acgaaaaaag atttcagaga taaaacacta gaagttacct   2460
   attctccacc taaatctctg aaaaatggag aaaccctctg actagtccat gtcaaatttt   2520
   actaaaagtc tttttgttta gatttatttt cctgcagcat cttctgcaaa atgtactata   2580
   tagtcagctt gctttgaggc tagtaaaaag atatttttct aaacagattg gagttggcat   2640
   ataaacaaat acgttttctc actaatgaca gtccatgatt cggaaatttt aagcccatga   2700
   atcagccgcg gtcttaccac ggtgatgcct gtgtgccgag agatgggact gtgcggccag   2760
   atatgcacag ataaatattt ggcttgtgta ttccatataa aattgcagtg catattatac   2820
   atccctgtga gccagatgct gaatagatat tttcctatta tttcagtcct ttataaaagg   2880
   aaaaataaac cagtttttaa atgtatgtat ataattctcc cccatttaca atccttcatg   2940
   tattacatag aaggattgct tttttaaaaa tatactgcgg gttggaaagg gatatttaat   3000
   ctttgagaaa ctattttaga aaatatgttt gtagaacaat tatttttgaa aaagatttaa   3060
   agcaataaca agaaggaagg cgagaggagc agaacatttt ggtctagggt ggtttctttt   3120
   taaaccattt tttcttgtta atttacagtt aaacctaggg gacaatccgg attggccctc   3180
   ccccttttgt aaataaccca ggaaatgtaa taaattcatt atcttagggt gatctgccct   3240
   gccaatcaga ctttggggag atggcgattt gattacagac gttcgggggg gtggggggct   3300
   tgcagtttgt tttggagata atacagtttc ctgctatctg ccgctcctat ctagaggcaa   3360
   cacttaagca gtaattgctg ttgcttgttg tcaaaatttg atcattgtta aaggattgct   3420
   gcaaataaat acactttaat ttcagtcaaa aa                                 3452
<210> 4
   <211> 2091
   <212> DNA
   <213> Homo sapiens
<400> 4
   atgagtgaag gggcagccgc tgcctcgcca cctggtgccg cttcggcagc cgccgcctcg     60
   gccgaggagg gcaccgcggc ggctgcggcg gcggcagcgg cgggcggggg cccggacggc    120
   ggcggcgaag gggcggccga gcccccccgg gagttacgct gtagcgactg catcgtgtgg    180
   aaccggcagc agacgtggct gtgcgtggta cctctgttca tcggcttcat cggcctgggg    240
   ctcagcctca tgcttctcaa atggatcgtg gtgggctccg tcaaggagta cgtgcccacc    300
   gacctagtgg actccaaggg gatgggccag gaccccttct tcctctccaa gcccagctct    360
   ttccccaagg ccatggagac caccaccact accacttcca ccacgtcccc cgccaccccc    420
   tccgccgggg gtgccgcctc ctccaggacg cccaaccgga ttagcactcg cctgaccacc    480
   atcacgcggg cgcccactcg cttccccggg caccgggtgc ccatccgggc cagcccgcgc    540
   tccaccacag cacggaacac tgcggcccct gcgacggtcc cgtccaccac ggccccgttc    600
   ttcagtagca gcacgctggg ctcccgaccc ccggtgccag gaactccaag tacccaggca    660
   atgccctcct ggcctactgc ggcatacgct acctcctcct accttcacga ttctactccc    720
   tcctggaccc tgtctccctt tcaggatgct gcctcctctt cttcctcttc ttcctcctcc    780
   gctaccacca ccacaccaga aactagcacc agccccaaat ttcatacgac gacatattcc    840
   acagagcgat ccgagcactt caaaccctgc cgagacaagg accttgcata ctgtctcaat    900
   gatggcgagt gctttgtgat cgaaaccctg accggatccc ataaacactg tcggtgcaaa    960
   gaaggctacc aaggagtccg ttgtgatcaa tttctgccga aaactgattc catcttatcg   1020
   gatccaacag accacttggg gattgaattc atggagagtg aagaagttta tcaaaggcag   1080
   gtgctgtcaa tttcatgtat catctttgga attgtcatcg tgggcatgtt ctgtgcagca   1140
   ttctacttca aaagcaagaa acaagctaaa caaatccaag agcagctgaa agtgccacaa   1200
   aatggtaaaa gctacagtct caaagcatcc agcacaatgg caaagtcaga gaacttggtg   1260
   aagagccatg tccagctgca aaattattca aaggtggaaa ggcatcctgt gactgcattg   1320
   gagaaaatga tggagtcaag ttttgtcggc ccccagtcat tccctgaggt cccttctcct   1380
   gacagaggaa gccagtctgt caaacaccac aggagtctat cctcttgctg cagcccaggg   1440
   caaagaagtg gcatgctcca taggaatgcc ttcagaagga cacccccgtc accccgaagt   1500
   aggctaggtg gaattgtggg accagcatat cagcaactcg aagaatcaag gatcccagac   1560
   caggatacga taccttgcca agggtattca tccagtggtt taaaaaccca acgaaataca   1620
   tcaataaata tgcaactgcc ttcaagagag acaaacccct attttaatag cttggagcaa   1680
   aaggacctgg tgggctattc atccacaagg gccagttctg tgcccatcat cccttcagtg   1740
   ggtttagagg aaacctgcct gcaaatgcca gggatttctg aagtcaaaag catcaaatgg   1800
   tgcaaaaact cctattcagc tgacgttgtc aatgtgagta ttccagtcag cgattgtctt   1860
   atagcagaac aacaagaagt gaaaatattg ctagaaactg tccaggagca gatccgaatt   1920
   ctgactgatg ccagacggtc agaagactac gaactggcca gcgtagaaac cgaggacagt   1980
   gcaagcgaaa acacagcctt tctccccctg agtcccacag ccaaatcaga acgagaggcg   2040
   caatttgtct taagaaatga aatacaaaga gactctgcat tgaccaagtg a             2091
<210> 5
   <211> 2608
   <212> DNA
   <213> Homo sapiens
<400> 5
   aaagggtggg gggcgggggt agggggagcc aatcccggca gcgccaaaga ggggaggagg 60
   cggtgacagc cgcggggagg gggcgggagg agagaggcag ctcggctcgg ctccgcgctc 120
   agctccgctc tgcctccggc tctgcgctca cctgctgcct agtgttccct ctcctgctcc 180
   aggacctccg ggtagacctc agaccccggg cccattccca gactcagcct cagcccggac 240
   ttccccagcc ccgacagcac agtaggccgc cagggggcgc cgtgtgagcg ccctatcccg 300
   gccacccggc gccccctccc acggcccggg cgggagcggg gcgccggggg ccatgcgggg 360
   ccagggccgc aaggagagtt tgtccgattc ccgagacctg gacggctcct acgaccagct 420
   cacgggccac cctccagggc ccactaaaaa agcgctgaag cagcgattcc tcaagctgct 480
   gccgtgctgc gggccccaag ccctgccctc agtcagtgaa attggccggg tcttccgctt 540
   tctcggtgac agttcgctcc cttcagcatt agccgcccca gcctccctcc gcccccacag 600
   accccgcctg ctggacccag acagcgtgga cgatgaattt gaattgtcca ccgtgtgtca 660
   ccggcctgag ggtctggagc agctgcagga gcaaaccaaa ttcacgcgca aggagttgca 720
   ggtcctgtac cggggcttca agaacgaatg tcccagcgga attgtcaatg aggagaactt 780
   caagcagatt tactcccagt tctttcctca aggagactcc agcacctatg ccacttttct 840
   cttcaatgcc tttgacacca accatgatgg ctcggtcagt tttgaggact ttgtggctgg 900
   tttgtccgtg attcttcggg gaactgtaga tgacaggctt aattgggcct tcaacctgta 960
   tgaccttaac aaggacggct gcatcaccaa ggaggaaatg cttgacatca tgaagtccat 1020
   ctatgacatg atgggcaagt acacgtaccc tgcactccgg gaggaggccc caagggaaca 1080
   cgtggagagc ttcttccaga agatggacag aaacaaggat ggtgtggtga ccattgagga 1140
   attcattgag tcttgtcaaa aggatgagaa catcatgagg tccatgcagc tctttgacaa 1200
   tgtcatctag cccccaggag agggggtcag tgtttcctgg ggggaccatg ctctaaccct 1260
   agtccaggcg gacctcaccc ttctcttccc aggtctatcc tcatcctacg cctccctggg 1320
   ggctggaggg atccaagagc ttggggattc agtagtccag atctctggag ctgaaggggc 1380
   cagagagtgg gcagagtgca tctcgggggg tgttcccaac tcccaccagc tctcaccccc 1440
   ttcctgcctg acacccagtg ttgagagtgc ccctcctgta ggaattgagc ggttccccac 1500
   ctcctacccc tactctagaa acacactaga cagatgtctc ctgctatggt gcttccccca 1560
   tccctgacct cataaacatt tcccctaaga ctcccctctc agagagaatg ctccattctt 1620
   ggcactggct ggcttctcag accagccatt gagagccctg tgggaggggg acaagaatgt 1680
   atagggagaa atcttgggcc tgagtcaatg gataggtcct aggaggtggc tggggttgag 1740
   aatagaaggg cctggacaga ttatgattgc tcaggcatac caggttatag ctccaagttc 1800
   cacaggtctg ctaccacagg ccatcaaaat ataagtttcc aggctttgca gaagaccttg 1860
   tctccttaga aatgccccag aaattttcca caccctcctc ggtatccatg gagagcctgg 1920
   ggccagatat ctggctcatc tctggcattg cttcctctcc ttccttcctg catgtgttgg 1980
   tggtggttgt ggtgggggaa tgtggatggg ggatgtcctg gctgatgcct gccaaaattt 2040
   catcccaccc tccttgctta tcgtccctgt tttgagggct atgacttgag tttttgtttc 2100
   ccatgttctc tatagacttg ggaccttcct gaacttgggg cctatcactc cccacagtgg 2160
   atgccttaga agggagaggg aaggagggag gcaggcatag catctgaacc cagtgtgggg 2220
   gcattcacta gaatcttcaa tcaacctggg ctctccccac cccaccccag ataacctcct 2280
   cagttcccta gggtctcttc ttgcttgact caatctaccc agagatgccc cttagcacac 2340
   ctagagggca gggaccatag gacccaggtt ccaaccccat tgtcagcacc ccagccatgc 2400
   ggccacccct tagcacacct gctcgtccca tttagcttac cctcccagtt ggccagaatc 2460
   tgaggggaga gcccccagag agcccccttc cccatcagaa gactgttgac tgctttgcat 2520
   tttgggctct tctatatatt ttgtaaagta agaaatatac cagatctaat aaaacacaat 2580
   ggctatgcaa aaaaaaaaaa aaaaaaaa 2608
<210> 6
   <211> 2100
   <212> DNA
   <213> Homo sapiens
<400> 6
   gtcccttctc tctttgcctg gaaagcttta gctggatagg ctggaaaggc agcagggtcc     60
   caggggctct agggttgggc agaggcataa tcacctccac tttgtatagt gtttgcctcg    120
   tgcgaagtgc actccacccc acacttggcc ccacagcctc gctgtgcacc tccttgcatc    180
   ggccggacgg gggcgggggg tgcgggcgga ggacggggaa ggaggctcct gtgtcccctc    240
   ttctcgcccc tgcacacact cgcgccacgg cgacccatgt tgatcaccgg agtttgggaa    300
   gtcagagggg agccctctgc tttcaggcct gggttccttc tctaagatat gcccacttag    360
   gggcctgtga ctcctccaga atgagtgtac cacactcggg acaccagaag cgagagggga    420
   cacaggctct gacctcgaga gcacgtcagc tctggagacc cgcggcccca gcctcgtgtg    480
   cctccgcaca gggcagcttc agcactgcga acagcgccag caccgcggac agcgcgctcc    540
   cacctcgccc ccggaggcca cgggctcggg ccgtgttcag caccgcggac agcgccagca    600
   actccgaggg tctcctggcc tccagcccgc cgtctctgac ctccctgccc cgccctcgac    660
   tccttctcaa actttgggat gccccagggg aggaagaggg cggcaggctg tgtgtggagt    720
   cttggcacgg cgtacattac acagcccgat caaaagccag cccgttcacc tcgaccaagc    780
   cagcatccac caaagggcca gaagtttacc aggccgctca ccgcgtgggc ttccattctc    840
   ctacctgcaa gctgcgcgag ttaaggaggg aaagcacgtg gcacagggcc tggcttctgg    900
   cgaggagccc accagagatg caaaggtgag gctgaaaccc tttaaactta aaaacaagaa    960
   gatacaaaca attggaacaa ctggggacat gggtgctatg tggagggcag gacgcctggc   1020
   ttcccatcag ggctgcagag gcggccgcgg ctggctccct ctcccgggtg ctgctggttg   1080
   aacttggaga gcaccctggc tcctgagggt ttaccgacca cagggtcagg ccggggcagg   1140
   gattaccacc ttacagatgc atagcctgag gccagtgagg acccacgctg tgctcagtgc   1200
   ccctggccgg taagtggtag gatttttatg tgaacgctgg gttcctttca cacagcgttt   1260
   ttcatgtgca aagtcatagg taattgtgtg actggtctct gagagaaagc atccaagccc   1320
   agaggggtcc ttactcctca ggctctgtcc ctccagactc tctcttattg cccctgccct   1380
   ctctggcctc tgcctgccac tggctacact gcatggggca gcagcatgcc caggttggag   1440
   cctgcctggg atggccacat tccctccctt gtccccaagc tcctgctggt actcagggct   1500
   tgaagcatag gcattctcct gtctgtggct gccacagtcg tctaggggtc tgtgtgggaa   1560
   tcaccagagt catgttgcct ggagacacat ggtcaggaga cccctcctga gtgggggtct   1620
   gggggtgcct tggccgcctg gcctctgcct gccactgccc cagactggct gcctcctgtc 1680
   tgctttccat cattctaaaa tgtaaattga atagtactcc tccccttcca gaagtgccag 1740
   ggaccatgtc taggctctgc agcctggtat caggggccat tcctggctgg atccttccag 1800
   ctatcctgta tgctccctag gcctgtaccc ggtactccaa gcccctccct gtgcctggat 1860
   ggccgagctg tcctctctgc tggtggttac tctgagcccc tccctgtgcc tggatggccg 1920
   agccgccatg tcctctctgc tggtggttac tccgagcccc tccctgtgcc tggatggctg 1980
   agctgccatg tcctctctgc tggtggttac tccgagcccc tccctgtgcc tggatggccg 2040
   agctgccatg tcctctcggt gcccttagcc actcactagt gcacatgcat catccctgct 2100
<210> 7
   <211> 2501
   <212> DNA
   <213> Homo sapiens
<400> 7
   aaagtgtata tggctaatcc tctcaagggc attccttcat gccttccctt tgctctacag 60
   gacccagaca gggcagggca tggagcaggc acacagtccg actgtctggc tggactcctg 120
   acccttacca tttggtccaa ctgcattgtg ctgagctctt gagccaccag tttatagggt 180
   tttgacacag attatgcctt gtctgtgtct ttctctcact cgcccatcca tccatccatc 240
   catccatcca tccatccatc catccatgat taggaaagac atctatattt gttgagtaca 300
   tgtttattaa tgtgtagaga acacagcaga gttggagtct cctaaaacat gacttgcact 360
   ggctggtgcc acctataggg gttcaactgc ttgattttct ttttcttcct tgtccctacc 420
   gccccccccg ctcccccgcc accatccctc tttgaataaa tgaagatgtt gaggttcttc 480
   tatcgaggtt ccgctcactg atgctagata tctaaacttc atgcagagaa gccatctaca 540
   gaggcaacag gccagggtgg gggcgagggg gagggcctga aagaagttgc ccccttctgt 600
   tgatctccga cccgcttgaa atctgaggca tctcctgggg tcaggaggct gccttaatat 660
   ccgcccccaa cactaaacat ttagcgcagg gcaaggagag gcaggcgagg gagggaggga 720
   aagggggaag cacatggccc tgctttggcc tgtttgcctc ccataataag agggaaagga 780
   atagaaaaca cccaggcaca acataaatat ttcctattat ggagaaacta aagtcacaat 840
   ggaatctgtc atgcttgtga tatgagagat ggacaatatt tcggggctgc tgccatgctg 900
   gcacctgaat ggagtttata ttttgtttat ggagctgaat ccgaagcctt ttattttttt 960
   ggctcggcct gtgtcattct tcatggaagc tcgaaatggc cgcggagcag ctcaatattc 1020
   catttatttt acttataaaa gaaattcaaa ggctgaaaag atgaagtaat tgacttagtg 1080
   ggatgacagt taaacccact aaatttaatc ttatcctctc aagtacaaca tatcgcgctt 1140
   acaaattatc gccagccacc aggcagcccc gcgctcctca cctaatgcaa atggagcgga 1200
   ggcctctgtt attgtattat ctgactttac tatcagcatt aatccggctt agcccgctta 1260
   atgatgccac ttgtggcact ccgaaggcag cggagaagtc tataaactac ttccattaaa 1320
   atgcaaatcg ggcccgctga gaaaacaagt gacacaaaaa gtgggcgccg cgcggccgcc 1380
   gcccgcgatg gcgccgatgg ctgatgccgc gattacgccg gcgggggcgg cccgcgcgcc 1440
   acgaacgtgc ccgacaagcc gcgccgctta atttctgaag agatttatat tttttttgtt 1500
   gtcaggaaat gaacaaatac ttatcaatgt aaaaactgct atctccgatc tccccgccgg 1560
   gtttttcata ctgatattct ttgcaccccg taacacagga aatgaaaatc ttctccgcct 1620
   ctcacgtcgc cgctgcgcct tcaaatcctc tgcgagcaga ctggcctccc cccaccggcc 1680
   ccctcccggg ctcccctccc cggtaaacag aaaagaaaac ttctgtgggc gaaggaggga 1740
   gaaattccaa ggctctggct ttccccagcc gacttgggtt tcggggcagc gcgggctccc 1800
   ttctttccag cgcaggaaga aggggtgagg gaggaggggc cggggtgctg gcactcaacg 1860
   gtaacatctg ttttacttaa gcagctcgat ttcgttttag gatgaaaaag tattcccttt 1920
   cagcatgagg aatcgtgggc cctggggctt gtattgattc agaacaaggc ctcataaaac 1980
   actagcaggt gatttagttt catattttga ttcctttgtt tagacagttt ctctcaggtt 2040
   ctcctgtgtc ggcccgggga ccccagcatc cctcctttga tgttggaggg acacccgggc 2100
   cgtcgagctc gccccgctct tgtgggtgac tttgtgctct tgagggcgca gcatttccca 2160
   aggttctccc ccaccctcac ccggcgagcc caggctctgc ggtcaggctc tcaaatcctg 2220
   caaggattga tggtcctcac agagggatca atactacatt ttaaatatta caataccttt 2280
   tattgagaca ctattacaac acaaatgttt taccctgtca cgggacccag ttaaaattgc 2340
   ttttgaccat cttattggtg ggcttccccg gccctctgtg tttcccactt ctgggaggag 2400
   tcactgtgct cccgggatgg gctggccccg cccaggaggc ctgggaagga ggaggatgca 2460
   gcctgggagg gctgtggggc acctggggag tgctgcagaa a 2501
<210> 8
   <211> 24
   <212> DNA
   <213> Artificial sequence
<220>
   <223> designed primer based on human gene
<400> 8
   gttcgggttg gtcgtttatc gttc 24
<210> 9
   <211> 22
   <212> DNA
   <213> Artificial Sequence
<220>
   <223> designed primer based on human gene
<400> 9
   cccacacact cgacccctaa cg 22
<210> 10
   <211> 21
   <212> DNA
   <213> Artificial Sequence
<220>
   <223> designed primer based on human gene
<400> 10
   agacgtattg gggcgagggg c 21
<210> 11
   <211> 21
   <212> DNA
   <213> Artificial Sequence
<220>
   <223> designed primer based on human gene
<400> 11
   ctccccgcga tcacacgcac g 21
<210> 12
   <211> 25
   <212> DNA
   <213> Artificial sequence
<220>
   <223> designed primer based on human gene
<400> 12
   tttcgcggag acgttaattt agttc 25
<210> 13
   <211> 24
   <212> DNA
   <213> Artificial Sequence
<220>
   <223> designed primer based on human gene
<400> 13
   aaacgcaaaa cgtatacaac gccg 24
<210> 14
   <211> 24
   <212> DNA
   <213> Artificial sequence
<220>
   <223> designed primer based on human gene
<400> 14
   gagggttggg aaattgcggt tttc 24
<210> 15
   <211> 21
   <212> DNA
   <213> Artificial Sequence
<220>
   <223> designed primer based on human gene
<400> 15
   ctcctcgcgc tacgtcatcc g 21
<210> 16
   <211> 22
   <212> DNA
   <213> Artificial Sequence
<220>
   <223> designed primer based on human gene
<400> 16
   gcgggtcggt attagttcgg tc 22
<210> 17
   <211> 25
   <212> DNA
   <213> Artificial Sequence
<220>
   <223> designed primer based on human gene
<400> 17
   ctacgacgta taaaacccgt aaacg 25
<210> 18
   <211> 24
   <212> DNA
   <213> Artificial Sequence
<220>
   <223> designed primer based on human gene
<400> 18
   ggggttatgt aggcgcgtta tttc 24
<210> 19
   <211> 25
   <212> DNA
   <213> Artificial Sequence
<220>
   <223> designed primer based on human gene
<400> 19
   tacgaataca cgctcataaa aaccg 25
<210> 20
   <211> 21
   <212> DNA
   <213> Artificial Sequence
<220>
   <223> designed primer based on human gene
<400> 20
   gggattcggc gcgtaggagg c 21
<210> 21
   <211> 24
   <212> DNA
   <213> Artificial Sequence
<220>
   <223> designed primer based on human gene
<400> 21
   caacgtaact cccgaaaaaa ctcg 24
<210> 22
   <211> 24
   <212> DNA
   <213> Artificial Sequence
<220>
   <223> designed primer based on human gene
<400> 22
   gtcgtttttt gatcgatcgg aggc 24
<210> 23
   <211> 25
   <212> DNA
   <213> Artificial Sequence
<220>
   <223> designed primer based on human gene
<400> 23
   aaaccgcgac aaaaacataa aaccg 25
<210> 24
   <211> 26
   <212> DNA
   <213> Artificial Sequence
<220>
   <223> designed primer based on human gene
<400> 24
   ttcgtttttc ggttttattc gatgtc 26
<210> 25
   <211> 26
   <212> DNA
   <213> Artificial Sequence
<220>
   <223> designed primer based on human gene
<400> 25
   actaaacgaa tctaaattaa tccccg 26
<210> 26
   <211> 26
   <212> DNA
   <213> Artificial Sequence
<220>
   <223> designed primer based on human gene
<400> 26
   gatggttatt ttcgaggttt attagc 26
<210> 27
   <211> 25
   <212> DNA
   <213> Artificial sequence
<220>
   <223> designed primer based on human gene
<400> 27
   cctccctttc taaaacgaaa atacg 25
<210> 28
   <211> 29
   <212> DNA
   <213> Artificial Sequence
<220>
   <223> designed primer based on human gene
<400> 28
   agtataatat atcgcgttta taaattatc 29
<210> 29
   <211> 23
   <212> DNA
   <213> Artificial Sequence
<220>
   <223> designed primer based on human gene
<400> 29
   aacgacgcga cttatcgaac acg 23
<210> 30
   <211> 27
   <212> DNA
   <213> Artificial Sequence
<220>
   <223> designed primer based on human gene
<400> 30
   gcgtttttta tttaatgtaa atggagc 27
<210> 31
   <211> 24
   <212> DNA
   <213> Artificial Sequence
<220>
   <223> designed primer based on human gene
<400> 31
   taatcgcgac atcaaccatc gacg 24
<210> 32
   <211> 25
   <212> DNA
   <213> Artificial Sequence
<220>
   <223> designed primer based on human gene
<400> 32
   gagagtacgt tagttttgga gattc 25
<210> 33
   <211> 24
   <212> DNA
   <213> Artificial Sequence
<220>
   <223> designed primer based on human gene
<400> 33
   cacgtacttt ccctccttaa ctcg 24
<210> 34
   <211> 26
   <212> DNA
   <213> Artificial Sequence
<220>
   <223> designed primer based on human gene
<400> 34
   ttagtattgc gaatagcgtt agtatc 26
<210> 35
   <211> 29
   <212> DNA
   <213> Artificial Sequence
<220>
   <223> designed primer based on human gene
<400> 35
   aataaatact aacttaatcg aaataaacg 29